(19) Europäisches Patentamt
European Patent Office
Office européen des brevets

(11) EP 1 413 884 A1

(12) **EUROPEAN PATENT APPLICATION**
published in accordance with Art. 158(3) EPC

(43) Date of publication:
**28.04.2004 Bulletin 2004/18**

(21) Application number: **02741407.7**

(22) Date of filing: **03.07.2002**

(51) Int Cl.$^7$: **G01N 33/50**, G01N 33/15, C12N 15/09, C07K 14/47, A61K 38/00, A61K 39/395, A61K 45/00, A61P 11/00, A61P 11/06

(86) International application number:
**PCT/JP2002/006730**

(87) International publication number:
**WO 2003/005024 (16.01.2003 Gazette 2003/03)**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **04.07.2001 JP 2001203036**

(71) Applicant: **Takeda Chemical Industries, Ltd.**
**Osaka-shi, Osaka 541-0045 (JP)**

(72) Inventors:
- **NAKANISHI, Atsushi**
  **Tsukuba-shi, Ibaraki 305-0025 (JP)**
- **MORITA, Shigeru**
  **Tsukuba-shi, Ibaraki 300-3261 (JP)**

(74) Representative: **Rickard, Timothy Mark Adrian**
**Takeda Euro IP Department,**
**11-12 Charles II Street**
**London SW1Y 4QU (GB)**

(54) **USE OF DISEASE-ASSOCIATED GENE**

(57) The present invention has an object to provide a method of screening a compound capable of inhibiting the activities of the disease-associated gene product, compounds obtainable by the screening method, etc. The compounds or a salt thereof that inhibits the activity of the protein having an amino acid sequence identical or substantially identical with the amino acid sequence represented by SEQ ID NO: 1, or a neutralizing antibody that inhibits the activity of that protein may be used as a prophylactic and/or therapeutic agent for various diseases, for example, pulmonary/thoracic diseases and respiratory diseases accompanied by pulmonary/airway inflammation [e.g. chronic obstructive pulmonary diseases (such as chronic bronchitis, pulmonary emphysema, diffuse panbronchiolitis, intrinsic asthma), bronchial asthma, cystis fibrosis, hypersensitivity pneumonitis, pulmonary fibrosis, etc.], inflammatory bowel diseases, allergic conjunctivitis, and so on.



Printed by Jouve, 75001 PARIS (FR)

## Description

### FIELD OF THE INVENTION

The present invention relates to use of a disease-associated gene. More specifically, the present invention relates to an antisense polynucleotide to a disease-associated gene that is useful as a diagnostic marker for chronic obstructive pulmonary diseases, etc.; an antibody to the product of the disease-associated gene; a method of screening for drugs using the disease-associated gene product; a diagnostic agent comprising a DNA of the disease-associated gene that is useful as a diagnostic marker for chronic obstructive pulmonary diseases, etc.; and so forth.

### BACKGROUND ART

It is considered that chronic obstructive pulmonary diseases (chronic bronchitis, pulmonary emphysema, diffuse panbronchiolitis, intrinsic asthma, etc.) will become major diseases in respiratory diseases with the aging of cigarette smoker generations and the prolongation of the average life span.

Further, Bronchial asthma is a chronic inflammatory disease of airways showing respiratory stenosis, in which symptoms such as paroxysmal dyspnea, stridor, cough, etc. are observed. Many cells such as respiratory epithelial cells, mast cells, eosinophils, T lymphocytes, etc. take part in its onset and development. One of the most important characteristics of bronchial asthma is that airways are hyperresponsive to irritants (airway hyperresponsiveness). This airway hyperresponsiveness is attributable to respiratory inflammation caused chiefly due to desquamation of respiratory epithelial cells by neurotransmitters secreted from the cells such as eosinophils, etc., infiltrated into airways, and it is also considered that genetic factors or environmental factors will affect the airway hyperresponsiveness complicatedly.

When the inflammatory reaction of airways is triggered by external irritants (allergens, exhausts) or viral infection, adhesion molecules including VCAM-1, ICAM-1 and the like are expressed on respiratory epithelial cells or on capillary endothelial cells around the bronchi [J. Allergy Clin. Immunol., 96, 941 (1995)] to produce cytokines or chemotactic substances. In patients with bronchial asthma, the function of Th2 type helper T cells is accentuated to increase the production of Th2 type cytokines such as IL-3, IL-4, IL-5, IL-13, GM-CSF, etc., or chemokines such as eotaxin, RANTES, etc. IL-4 or IL-13 has an activity of inducing IgE production, and IL-3 or IL-4 has an activity of inducing the growth of mast cells. Furthermore, eosinophils are differentiated and proliferated by the action of IL-5, GM-CSF, etc. and mediated by eotaxin or RANTES to infiltrate into the airways [Allergy Asthma Proc., 20, 141 (1999)].

Epithelial cells that cover the tracheal and bronchial mucosa not only have the barrier function to prevent direct transmittance of external irritants to submucosal tissues and the function to excrete secretions or foreign matters, but also control contraction of the trachea by secreted epithelium-derived smooth muscle relaxing factors, etc. Eosinophils infiltrated into the airways of patient with bronchial asthma release through degranulation intracellular granule proteins such as activated MBP (major basic protein), ECP (eosinophil cationic protein), etc. [Compr. Ther., 20, 651 (1994)]. By the cytotoxic action of these granular proteins, the desquamation/damages of epithelial cells occur. The desquamation of epithelial cells leads to exposure of sensory nerve terminals, accentuation of epithelial permeability and loss of epithelium-derived smooth muscle relaxing factors. Also, leukotriene C4 (LTC4) or platelet activating factor (PAF) produced by eosinophils accentuate tension of bronchial smooth muscle. It is considered that when the foregoing changes are repeated to make it chronic, the bronchial walls would be thickened to cause airway hyperresponsiveness. However, there is no report relating to a gene, expression of which is localized in the lesion of lung/bronchi and which is associated with the onset of chronic obstructive pulmonary disease and airway hyperresponsiveness.

On the other hand, human-derived CLCA (Cl-channel, $Ca^{2+}$-activated) DNA and protein belong to a chloride channel protein family (FEBS Letters, 455, 295 (1999), but there is no report that they would be associated with chronic obstructive pulmonary disease (COPD) and bronchial asthma.

### DISCLOSURE OF THE INVENTION

The present invention provides a method of screening for compounds that inhibit the activity of a protein whose expression is increased in the lung and/or the bronchi of chronic obstructive pulmonary disease patients and asthma patients; compounds obtainable by the screening method; and so forth.

As a result of intensive and extensive researches toward the solution of the above problems, the present inventors have found a cDNA the expression of which is remarkably increased in induced sputum from chronic obstructive pulmonary disease patients. The present inventors have made further examination based on this finding and finally achieved the present invention.

That is, the present invention relates to the following features.

(1) A method of screening for a compound or a salt thereof that inhibits the activity of a protein comprising the same or substantially the same amino acid sequence as represented by SEQ ID NO: 1, a partial peptide thereof, or a salt thereof, which comprises using a protein comprising the same or substantially the same amino acid sequence as represented by SEQ ID NO: 1, a partial peptide thereof, or a salt thereof.

(2) A kit for screening a compound or a salt thereof that inhibits the activity of a protein comprising the same or substantially the same amino acid sequence as represented by SEQ ID NO: 1, a partial peptide thereof, or a salt thereof, which contains a protein comprising the same or substantially the same amino acid sequence as represented by SEQ ID NO: 1, a partial peptide thereof, or a salt thereof.

(3) A compound or a salt thereof that inhibits the activity of a protein comprising the same or substantially the same amino acid sequence as represented by SEQ ID NO: 1, a partial peptide thereof, or a salt thereof, wherein said compound or salt thereof is obtainable by using the screening method according to (1) or the screening kit according to (2).

(4) A medicine comprising the compound or salt thereof according to (3).

(5) The medicine according to (4), which is a prophylactic and/or therapeutic agent for respiratory diseases.

(6) The medicine according to (5), wherein the respiratory disease is a chronic obstructive pulmonary disease or bronchial asthma.

(7) A method of screening for a compound or a salt thereof that inhibits the expression of the gene for a protein comprising the same or substantially the same amino acid sequence as represented by SEQ ID NO: 1, which comprises using a polynucleotide comprising a polynucleotide encoding said protein or a partial peptide thereof..

(8) A screening kit for a compound or a salt thereof that inhibits the expression of the gene for a protein comprising the same or substantially the same amino acid sequence as represented by SEQ ID NO: 1, which contains a polynucleotide comprising a polynucleotide encoding said protein or a partial peptide thereof.

(9) A compound or a salt thereof that inhibits the expression of the gene for a protein comprising the same or substantially the same amino acid sequence as represented by SEQ ID NO: 1, wherein said compound or salt thereof is obtainable by using the screening method according to (7) or the screening kit according to (8).

(10) A medicine comprising the compound or salt thereof according to (9).

(11) The medicine according to (10), which is a prophylactic and/or therapeutic agent for respiratory diseases.

(12) The medicine according to (11), wherein the respiratory disease is a chronic obstructive pulmonary disease or bronchial asthma.

(13) An antisense polynucleotide comprising a nucleotide sequence, or a part thereof, that is complementary or substantially complementary to the nucleotide sequence of a DNA encoding a protein comprising the same or substantially the same amino acid sequence as represented by SEQ ID NO: 1 or a partial peptide of said protein.

(14) A medicine comprising the antisense polynucleotide according to (13).

(15) The medicine according to (14), which is a prophylactic and/or therapeutic agent for respiratory diseases.

(16) The medicine according to (15), wherein the respiratory disease is a chronic obstructive pulmonary disease or bronchial asthma.

(17) An antibody to a protein comprising the same or substantially the same amino acid sequence as represented by SEQ ID NO: 1, a partial peptide thereof, or a salt thereof.

(18) A method of quantitatively determining a protein comprising the same or substantially the same amino acid sequence as represented by SEQ ID NO: 1, a partial peptide thereof, or a salt thereof, which comprises using the antibody according to (17).

(19) A medicine comprising the antibody according to (17).

(20) A diagnostic agent comprising the antibody according to (14).

(21) The medicine according to (19), which is a prophylactic and/or therapeutic agent for respiratory diseases.

(22) The medicine according to (21), wherein the respiratory disease is a chronic obstructive pulmonary disease or bronchial asthma.

(23) The diagnostic agent according to (20), which is a diagnostic agent for respiratory diseases.

(24) The diagnostic agent according to (23), wherein the respiratory disease is a chronic obstructive pulmonary disease or bronchial asthma.

(25) A method of diagnosing respiratory diseases, comprising using the quantitative determination method according to (18).

(26) A method of screening for a compound or a salt thereof that inhibits the production of a protein comprising the same or substantially the same amino acid sequence as represented by SEQ ID NO: 1, which comprises using the antibody according to (17).

(27) A kit for screening for a compound or a salt thereof that inhibits the production of a protein comprising the same or substantially the same amino acid sequence as represented by SEQ ID NO: 1, which contains the antibody according to (17).

(28) A compound or a salt thereof that inhibits the production of a protein comprising the same or substantially the same amino acid sequence as represented by SEQ ID NO: 1, wherein said compound or salt thereof is obtainable by using the screening method according to (26) or the screening kit according to (27).

(29) A medicine comprising the compound or salt thereof according to (28).

(30) The medicine according to (29), which is a prophylactic and/or therapeutic agent for respiratory diseases.

(31) The medicine according to (30), wherein the respiratory disease is a chronic obstructive pulmonary disease or bronchial asthma.

(32) A diagnostic agent comprising a DNA encoding a protein comprising the same or substantially the same amino acid sequence as represented by SEQ ID NO: 1 or a partial peptide thereof.

(33) The diagnostic agent according to (32), which is a diagnostic agent for respiratory diseases.

(34) The diagnostic agent according to (33), wherein the respiratory disease is a chronic obstructive pulmonary disease or bronchial asthma.

(35) A prophylactic and/or therapeutic agent for respiratory diseases, comprising a compound or a salt thereof that inhibits the activity of a protein comprising the same or substantially the same amino acid sequence as represented by SEQ ID NO: 1, a partial peptide thereof, or a salt thereof; a compound or a salt thereof that inhibits the expression of the gene for said protein; or a compound or a salt thereof that inhibits the production of said protein.

(36) A method of preventing and/or treating a respiratory disease, comprising administering to a mammal an effective amount of the compound or salt thereof according to (3), (9) or (28).

(37) A method of preventing and/or treating a respiratory disease, comprising administering to a mammal an effective amount of the antisense polynucleotide according to (13).

(38) A method of preventing and/or treating a respiratory disease, comprising administering to a mammal an effective amount of the antibody according to (17).

(39) A method of preventing and/or treating a respiratory disease, comprising administering to a mammal an effective amount of a compound or a salt thereof that inhibits the activity of a protein comprising the same or substantially the same amino acid sequence as represented by SEQ ID NO: 1, a partial peptide thereof, or a salt thereof; a compound or a salt thereof that inhibits the expression of the gene for said protein; or a compound or a salt thereof that inhibits the production of said protein.

(40) Use of the compound or salt thereof according to (3), (9) or (28) for producing a prophylactic and/or therapeutic agent for respiratory diseases.

(41) Use of the antisense polynucleotide according to (13) or a salt thereof for producing a prophylactic and/or therapeutic agent for respiratory diseases.

(42) Use of the antibody according to (17) for producing a prophylactic and/or therapeutic agent for respiratory diseases.

(43) Use of a compound or a salt thereof that inhibits the activity of a protein comprising the same or substantially the same amino acid sequence as represented by SEQ ID NO: 1, a partial peptide thereof, or a salt thereof; a compound or a salt thereof that inhibits the expression of the gene for said protein; or a compound or a salt thereof that inhibits the production of said protein; for producing a prophylactic and/or therapeutic agent for respiratory diseases.

## BRIEF DESCRIPTION OF THE DRAWINGS

Fig. 1 shows the expression levels of CLCA4 gene in non-smoking healthy individuals and COPD patients examined in Example 1.

Fig. 2 shows fluorescence intensities in the absence or presence of ionomycin stimulation in parent strain CHO cells and human CLCA4-expressiong CHO cells, examined in (3) in Example 2. In this Figure, the open column represents the result obtained with parent strain CHO cells, and the filled column represents the results obtained with human CLCA4-expressiong CHO cells.

Fig. 3 shows fluorescence intensities in human CLCA4-expressiong CHO cells when stimulated with ionomycin in the absence or presence of FNA, examined in (3) in Example 2.

## BEST MODE FOR CARRYING OUT THE INVENTION

The protein used in the present invention, which contains the same or substantially the same amino acid sequence as the amino acid sequence represented by SEQ ID NO: 1 (hereinafter sometimes referred to as the protein of the invention or the protein used in the present invention) may be any protein derived from any cells of human and other warm-blooded animals (e.g., guinea pig, rat, mouse, chicken, rabbit, swine, sheep, bovine, monkey, etc.) such as hepatocyte, splenocyte, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophage, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocyte or interstitial cells; or the corresponding precursor cells, stem cells, cancer cells, etc.; or any tissues where such cells are present, such as brain or any of brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc.; the proteins may also be synthetic proteins.

As the amino acid sequence having substantially the same amino acid sequence as that shown by SEQ ID NO: 1, there are amino acid sequences having at least about 60% homology, preferably at least about 70% homology, more preferably at least about 80% homology, much more preferably at least about 90% homology and most preferably at least about 95% homology, to the amino acid sequence shown by SEQ ID NO: 1.

Preferred examples of the protein containing substantially the same amino acid sequence as the amino acid sequence shown by SEQ ID NO: 1 include proteins having substantially the same amino acid sequence as the amino acid sequence shown by SEQ ID NO: 1 and having a property substantially equivalent to that of the protein having the amino acid sequence shown by SEQ ID NO: 1, etc.

As the substantially equivalent activity, there are, e.g., a chloride channel-like activity (calcium-dependent chloride channel-like activity), and the like. The substantially equivalent is used to mean that the nature of these properties is equivalent in property (e.g., physiologically or pharmacologically). Preferably, the chloride channel-like activity is equivalent (e.g., approximately 0.01 to 100 times, preferably approximately 0.1 to 10 times, more preferably 0.5 to twice), but differences in degree such as a level of these properties, quantitative factors such as a molecular weight, etc. may be present and allowable.

Measurement of the activities such as the chloride channel-like activity, etc. may be made by a modification of publicly known methods; for example, the measurement may be made by the method described in Genomics, 54, 200 (1998) or its modification.

Furthermore, examples of the protein used in the present invention include so-called muteins such as proteins containing 1) the amino acid sequence represented by SEQ ID NO: 1, of which at least 1 or 2 (preferably about 1 to about 30, more preferably about 1 to about 10 and most preferably several (1 to 5)) amino acids are deleted; 2) the amino acid sequence represented by SEQ ID NO: 1, to which at least 1 or 2 (preferably about 1 to about 30, more preferably about 1 to about 10 and most preferably several (1 to 5)) amino acids are added; 3) the amino acid sequence represented by SEQ ID NO: 1, in which at least 1 or 2 (preferably about 1 to about 30, more preferably about 1 to about 10 and most preferably several (1 to 5)) amino acids are inserted; 4) the amino acid sequence represented by SEQ ID NO: 1, in which at least 1 or 2 (preferably about 1 to about 30, more preferably about 1 to about 10 and most preferably several (1 to 5)) amino acids are substituted by other amino acids; or 5) a combination of the above amino acid sequences.

Throughout the specification, the proteins are represented in accordance with the conventional way of describing proteins, that is, the N-terminus (amino terminus) at the left hand and the C-terminus (carboxyl terminus) at the right hand. In the proteins used in the present invention including the protein containing the amino acid sequence shown by SEQ ID NO: 1, the C-terminus is usually in the form of a carboxyl group (-COOH) or a carboxylate (-COO') but may be in the form of an amide ($-CONH_2$) or an ester (-COOR).

Examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, $\alpha$-naphthyl, etc.; a $C_{7-14}$ aralkyl such as a phenyl-$C_{1-2}$ alkyl group, e.g., benzyl, phenethyl, etc.; an $\alpha$-naphthyl-$C_{1-2}$ alkyl group such as $\alpha$-naphthylmethyl, etc.; pivaloyloxymethyl and the like.

Where the protein used in the present invention-contains a carboxyl group (or a carboxylate) at a position other than the C-terminus, it may be amidated or esterified and such an amide or ester is also included within the protein of the present invention. The ester group may be the same group as that described with respect to the above C-terminal.

Furthermore, examples of the protein used in the present invention include variants of the above proteins, wherein the amino group at the N-terminus (e.g., methionine residue) of the protein is protected with a protecting group (e.g., a $C_{1-6}$ acyl group such as a $C_{1-6}$ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a $C_{1-6}$ acyl group such as a $C_{1-6}$ alkanoyl group, e.g., formyl group, acetyl group, etc.), or conjugated proteins such as glycoproteins having sugar chains.

Specific examples of the protein used in the present invention include a human-derived protein containing the amino acid sequence represented by SEQ ID NO: 1, and the like.

The partial peptide of the protein used in the present invention may be any peptide, so long as it is a partial peptide of the protein used in the present invention described above and preferably has the property equivalent to that of the protein used in the present invention described above.

Specifically, for the purpose of preparing the antibody of the present invention described later, examples of peptides which may be used include a peptide that has an amino acid sequence spanning from position 21 to position 917 of the amino acid sequence represented by SEQ ID NO: 1. Further, in the screening method and the screening kit of the present invention described later, examples of peptides which may be used preferably include a peptide that comprises a cell membrane binding site (an amino acid sequence spanning from position 1 to position 20 of the amino acid sequence represented by SEQ ID NO: 1) and has a part of an amino acid sequence spanning from position 21 to position 917 of the amino acid sequence represented by SEQ ID NO: 1. For example, a peptide having an amino acid sequence consisting of at least 20 or more, preferably 50 or more, more preferably 70 or more, still more preferably 100 and more, most preferably 200 or more amino acids in the constitutive amino acid sequence for the protein used in the present invention may be used.The partial peptides used in the present invention may be those containing the amino acid sequence, of which at least 1 or 2 (preferably about 1 to about 10 and more preferably several (1 to 5)) amino acids are deleted; to which at least 1 or 2 (preferably about 1 to about 10 and more preferably several (1 to 5)) amino acids are added; in which at least 1 or 2 (preferably about 1 to about 10 and more preferably several (1 to 5)) amino acids are inserted; or, in which at least 1 or 2 (preferably about 1 to about 10 and more preferably several (1 to 5)) amino acids are substituted by other amino acids.

In the partial peptide used in the present invention, the C-terminus is usually in the form of a carboxyl group (-COOH) or a carboxylate ($-COO^-$) but may be in the form of an amide ($-CONH_2$) or an ester (-COOR), as in the aforesaid proteins employed in the present invention.

The partial peptides used in the invention include those containing carboxyl groups (or carboxylates) other than at the C-terminus, those wherein the amino group of the N-terminal amino acid residue (e.g., methionine residue) is protected with a protecting group, those wherein the N-terminal region is cleaved in vivo and the glutamine thus formed is pyroglutaminated, those wherein a substituent on the side chain of an amino acid in the molecule is protected with a suitable protecting group, or conjugated proteins such as so-called glycoproteins to which sugar chains are bound, etc., as in the proteins used in the present invention described above.

The partial peptide used in the invention may also be used as an antigen for producing antibodies.

The protein or its partial peptide used in the present invention may be employed in the form of salts with physiologically acceptable acids (e.g., inorganic acids or organic acids) or bases (e.g., alkali metal salts), preferably in the form of physiologically acceptable acid addition salts. Examples of such salts are salts with inorganic acids (e.g., hydrochloric acid, phosphoric acid, hydrobromic acid, and sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The proteins or partial peptides used in the present invention, or salts thereof, may be manufactured by a publicly known method used to purify a protein from human or other warm-blooded animal cells or tissues described above. Alternatively, they may also be manufactured by culturing transformants containing DNAs encoding the proteins. Furthermore, they may also be manufactured by a modification of the methods for peptide synthesis, which will be later described.

Where these proteins or peptides are manufactured from human or mammalian tissues or cells, human or mammalian tissues or cells are homogenized, then extracted with an acid or the like, and the proteins or peptides are isolated and purified from the extract by a combination of chromatography techniques such as reverse phase chromatography, ion exchange chromatography, and the like.

To synthesize the proteins or partial peptides used in the present invention, or salts thereof or amides thereof, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenylhydroxymethyl)phenoxy resin, 4-(2',4'-dimethoxyphenylFmoc-aminoethyl) phenoxy resin, etc. Using these resins, amino acids, in which $\alpha$-amino groups and functional groups on the side chains are appropriately protected are condensed on the resin in the order of the sequence of the objective protein according to various condensation methods publicly known in the art. At the end of the reaction, the protein or partial peptide is excised from the resin and at the same time, the protecting groups are removed. Then, intramolecular disulfide bond-forming reaction is performed in a highly diluted solution to obtain the objective protein or partial peptide, or amides thereof.

For condensation of the protected amino acids described above, a variety of activation reagents for protein synthesis may be used, but carbodiimides are particularly preferably employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents that are known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to cancel any possible adverse affect on the subsequent reaction.

Examples of the protecting groups used to protect the starting amino groups include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples. of groups appropriately used for the esterification include a lower $C_{1-6}$ alkanoyl group, such as acetyl group, an aroyl group such as benzoyl group, and a group derived from carbonic acid such as benzyloxycarbonyl group and ethoxycarbonyl group. Examples of a group appropriately used for the etherification include benzyl group, tetrahydropyranyl group, t-butyl group, etc.

Examples of groups for protecting the phenolic hydroxyl group of tyrosine include Bzl, $Cl_2$-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting amino acids include the corresponding acid anhydrides, azides, activated esters (esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)). As the activated amino acids in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid or trifluoroacetic acid, or a mixture solution of these acids; a treatment with a base such as diisopropylethylamine, triethylamine, piperidine or piperazine; and reduction with sodium in liquid ammonia. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol or 1,4-butanedithiol, as well as by a treatment with an alkali such as a dilute sodium hydroxide solution and dilute ammonia.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the desired protein or partial peptide, for example, the α-carboxyl group of the carboxy terminal amino acid is first protected by amidation; the peptide (protein) chain is then extended from the amino group side to a desired length. Thereafter, a protein or partial peptide, in which only the protecting group of the N-terminal α-amino group of the peptide chain has been eliminated, and a protein or partial peptide, in which only the protecting group of the C-terminal carboxyl group has been eliminated are manufactured. The two proteins or peptides are condensed in a mixture of the solvents described above. The details of the condensation reaction are the same as described above. After the protected protein or peptide obtained by the condensation is purified, all the protecting groups are eliminated by the method described above to give the desired crude protein or peptide. This crude protein or peptide is purified by various known purification means. Lyophilization of the major fraction gives the amide of the desired protein or peptide.

To prepare the esterified protein or peptide, for example, the α-carboxyl group of the carboxy terminal amino acid is condensed with a desired alcohol to prepare the amino acid ester, which is followed by procedure similar to the preparation of the amidated protein or peptide above to give the desired esterified protein or peptide.

The partial peptide or salts thereof used in the present invention can be manufactured by publicly known methods for peptide synthesis, or by cleaving the protein used in the present invention with an appropriate peptidase. For the methods for peptide synthesis, for example, either solid phase synthesis or liquid phase synthesis may be used. That is, the partial peptide or amino acids that can construct the partial peptide used in the present invention are condensed with the remaining part. Where the product contains protecting groups, these protecting groups are removed to give the desired peptide. Publicly known methods for condensation and elimination of the protecting groups are described in 1) to 5) below.

1) M. Bodanszky & M.A. Ondetti: Peptide Synthesis, Interscience Publishers, New York (1966)
2) Schroeder & Luebke: The Peptide, Academic Press, New York (1965)
3) Nobuo Izumiya, et al.: *Peptide Gosei-no-Kiso to Jikken* (Basics and experiments of peptide synthesis), published by Maruzen Co. (1975)
4) Haruaki Yajima & Shunpei Sakakibara: *Seikagaku Jikken Koza* (Biochemical Experiment) 1, *Tanpakushitsu no Kagaku* (Chemistry of Proteins) IV, 205 (1977)
5) Haruaki Yajima ed.: *Zoku Iyakuhin no Kaihatsu* (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide used in the present invention. When the partial peptide obtained by the above methods is in a free form, the peptide can be converted into an appropriate salt by a publicly known method; conversely when the partial peptide is obtained in a salt form, it can be converted into a free form or other different salt form by a publicly known method.

The DNA encoding the protein used in the present invention may be any DNA so long as it contains the base sequence encoding the protein used in the present invention described above. The DNA may also be any one of genomic DNA, genomic DNA library, cDNA derived from the cells or tissues described above, cDNA library derived from the cells or tissues described above and synthetic DNA.

The vector to be used for the library may be any of bacteriophage, plasmid, cosmid, phagemid and the like. In addition, the DNA can be amplified by reverse transcriptase polymerase chain reaction (hereinafter abbreviated as RT-PCR) with total RNA or mRNA fraction prepared from the above-described cells or tissues.

As a DNA encoding the protein used in the present invention, a DNA comprising the nucleotide sequence represented by SEQ ID NO: 2 may be used, for example. Alternatively, any DNA may be used as long as it has a nucleotide sequence hybridizable with the nucleotide sequence represented by SEQ ID NO: 2 under stringent conditions and yet encodes a protein which has substantially the same nature as that of the protein used in the present invention.

As a DNA hybridizable with the nucleotide sequence represented by SEQ ID NO: 2 under stringent conditions, a DNA having a nucleotide sequence with about 60% or more, preferably about 70% or more, more preferably about 80% or more, particularly preferably about 90% or more, and most preferably about 95% or more homology to the nucleotide sequence represented by SEQ ID NO: 2 may be used, for example.The hybridization can be carried out by publicly known methods or by a modification thereof, for example, according to the method described in Molecular Cloning, 2nd Ed., J. Sambrook et al., Cold Spring Harbor Lab. Press, (1989). A commercially available library may also be used according to the instructions of the attached manufacturer's protocol. The hybridization can be carried out preferably under high stringent conditions.

The high stringent conditions used herein are, for example, those in a sodium concentration at about 19 mM to about 40 mM, preferably about 19 mM to about 20 mM at a temperature of about 50°C to about 70°C, preferably about 60°C to about 65°C. In particular, hybridization conditions in a sodium concentration at about 19 mM at a temperature of about 65°C are most preferred.

More specifically, as a DNA encoding a protein comprising the amino acid sequence represented by SEQ ID NO: 1, a DNA comprising the nucleotide sequence represented by SEQ ID NO: 2 may be used, for example.

The DNA encoding the partial peptide used in the present invention may be any DNA so long as it contains the base sequence encoding the partial peptide used in the present invention described above. The DNA may also be any of genomic DNA, genomic DNA library, cDNA derived from the cells and tissues described above, cDNA library derived from the cells and tissues described above and synthetic DNA.

As a DNA encoding a partial peptide used in the present invention, a DNA having a part of a DNA comprising the nucleotide sequence represented by SEQ ID NO: 2 may be used, for example. Alternatively, a DNA having a part of a DNA that comprises a nucleotide sequence hybridizable with the nucleotide sequence represented by SEQ ID NO: 2 under stringent conditions and yet encodes a protein having substantially the same activity as that of the protein of the present invention may be used.

The "DNA hybridizable with the nucleotide sequence represented by SEQ ID NO: 2" has the same meaning as described above.

Methods for the hybridization and the high stringent conditions that can be used are the same as those described above.

As a means of cloning a DNA that completely encodes the protein or partial peptide used in the present invention (hereinafter sometimes merely referred to as the protein of the present invention in describing the cloning and expression of the DNAs encoding the same), the DNA may be amplified either by PCR using synthetic DNA primers containing a part of the base sequence of the protein of the present invention, or the DNA inserted into an appropriate vector may be screened by hybridization with a labeled DNA fragment or synthetic DNA that encodes a part or entire region of the protein of the present invention. The hybridization can be carried out, for example, according to the method described in Molecular Cloning, 2nd (J. Sambrook et al., Cold Spring Harbor Lab. Press, 1989). Where the hybridization is carried out using commercially available library, the procedures may be conducted in accordance with the protocol described in the attached instructions.

Substitution of the base sequence of DNA can be effected by publicly known methods such as the ODA-LA PCR method, the Gapped duplex method or the Kunkel method, or modifications thereof by using publicly known kits available as Mutan™-super Express Km, Mutan™-K (both manufactured by Takara Shuzo Co., Ltd.), etc.

The cloned DNA encoding the protein can be used as it is depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector of the protein of the present invention can be manufactured, for example, by (a) excising the desired DNA fragment from the DNA encoding the protein of the present invention, (b) and then ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc.

Among them, CMV (cytomegalovirus) promoter or SRα promoter is preferably used. Where the host is bacteria of the genus Escherichia, preferred examples of the promoter include trp promoter, lac promoter, recA promoter, γPL promoter, lpp promoter, T7 promoter, etc. In the case of using bacteria of the genus Bacillus as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin prompter, P10 promoter, etc.

In addition to the foregoing examples, the expression vector may further optionally contain an enhancer, a splicing signal, a poly A addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as $Amp^r$), neomycin resistant gene (hereinafter sometimes abbreviated as $Neo^r$, G418 resistance), etc. In particular, when dhfr gene is used as the selection marker using dhfr gene-deficient Chinese hamster cells, selection can also be made on a thymidine free medium.

If necessary, a signal sequence that matches with a host is added to the N-terminus of the protein of the present invention. Examples of the signal sequence that can be used are PhoA signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

Using the vector containing the DNA encoding the protein of the present invention thus constructed, transformants can be manufactured.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichia coli K12 DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.
Examples of the bacteria belonging to the genus Bacillus include Bacillus subtilis MI114 [Gene, 24, 255 (1983)], 207-21 [Journal of Biochemistry, 95, 87 (1984)], etc.
Examples of yeast include Saccharomyces cereviseae AH22, AH22R$^-$, NA87-11A, DKD-5D, 20B-12, Schizosaccharomyces pombe NCYC1913, NCYC2036, Pichia pastoris KM71, etc.
Examples of insect cells include, for the virus AcNPV, Spodoptera frugiperda cell (Sf cell), MG1 cell derived from mid-intestine of Trichoplusia ni, High Five™ cell derived from egg of Trichoplusia ni, cells derived from Mamestra brassicae, cells derived from Estigmena acrea, etc.; and for the virus BmNPV, Bombyx mori N cell (BmN cell), etc. is used. Examples of the Sf cell which can be used are Sf9 cell (ATCC CRL1711), Sf21 cell (both cells are described in Vaughn, J. L. et al., In Vivo, 13, 213-217 (1977), etc.
As the insect, for example, a larva of Bombyx mori can be used [Maeda et al., Nature, 315, 592 (1985)].
Examples of animal cells include monkey cell COS-7, Vero, Chinese hamster cell CHO (hereinafter referred to as CHO cell), dhfr gene-deficient Chinese hamster cell CHO (hereinafter simply referred to as CHO (dhfr$^-$) cell), mouse L cell, mouse AtT-20, mouse myeloma cell, rat GH 3, human FL cell, etc.
Bacteria belonging to the genus Escherichia can be transformed, for example, by the method described in Proc. Natl. Acad. Sci. U.S.A., 69, 2110 (1972), Gene, 17, 107 (1982), etc.
Bacteria belonging to the genus Bacillus can be transformed, for example, by the method described in Molecular & General Genetics, 168, 111 (1979), etc.
Yeast can be transformed, for example, by the method described in Methods in Enzymology, 194, 182-187 (1991), Proc. Natl. Acad. Sci. U.S.A., 75, 1929 (1978), etc.
Insect cells or insects can be transformed, for example, according to the method described in Bio/Technology, 6, 47-55(1988), etc.
Animal cells can be transformed, for example, according to the method described in *Saibo Kogaku* (Cell Engineering), extra issue 8, *Shin Saibo Kogaku Jikken Protocol* (New Cell Engineering Experimental Protocol), 263-267 (1995)(published by Shujunsha), or Virology, 52, 456 (1973).
Thus, the transformants transformed with the expression vectors containing the DNAs encoding the protein can be obtained.
Where the host is bacteria belonging to the genus Escherichia or the genus Bacillus, the transformant can be appropriately cultured in a liquid medium which contains materials required for growth of the transformant such as carbon sources, nitrogen sources, inorganic materials, etc. Examples of the carbon sources include glucose, dextrin, soluble starch, sucrose, etc.; examples of the nitrogen sources include inorganic or organic materials such as ammonium salts, nitrate salts, corn steep liquor, peptone, casein, meat extract, soybean cake, potato extract, etc.; and, examples of the inorganic materials are calcium chloride, sodium dihydrogenphosphate, magnesium chloride, etc. In addition, yeast, vitamins, growth promoting factors etc. may also be added to the medium. Preferably, pH of the medium is adjusted to about 5 to about 8.
A preferred example of the medium for culturing the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.
Where the bacteria belonging to the genus Escherichia are used as the host, the transformant is usually cultivated at about 15°C to about 43°C for about 3 hours to about 24 hours. If necessary, the culture may be aerated or agitated.
Where the bacteria belonging to the genus Bacillus are used as the host, the transformant is cultured generally at about 30°C to about 40°C for about 6 hours to about 24 hours. If necessary, the culture can be aerated or agitated.
Where yeast is used as the host, the transformant is cultivated, for example, in Burkholder's minimal medium [Bostian, K. L. et al., Proc. Natl. Acad. Sci. U.S.A., 77, 4505 (1980)] or in SD medium supplemented with 0.5% Casamino acids [Bitter, G. A. et al., Proc. Natl. Acad. Sci. U.S.A., 81, 5330 (1984)]. Preferably, pH of the medium is adjusted to about 5 to about 8. In general, the transformant is cultivated at about 20°C to about 35°C for about 24 hours to about 72 hours. If necessary, the culture can be aerated or agitated.
Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary, the culture can be aerated or agitated.
Where animal cells are employed as the host, the transformant is cultured in, for example, MEM medium containing about 5% to about 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 hours to about 60 hours and, if necessary, the culture can be aerated or agitated.

As described above, the protein of the present invention can be produced intracellularly, on the cell membrane, or extracellularly of the transformant.

The protein of the present invention can be separated and purified from the culture described above by the following procedures.

When the protein of the present invention is extracted from the culture or cells, the transformant or cell is collected after culturing by a publicly known method and suspended in a appropriate buffer. The transformant or cell is then disrupted by publicly known methods such as ultrasonication, a treatment with lysozyme and/or freeze-thaw cycling, followed by centrifugation, filtration, etc. Thus, the crude extract of the protein can be obtained. The buffer used for the procedures may contain a protein modifier such as urea or guanidine hydrochloride, or a surfactant such as Triton X-100™, etc. When the protein is secreted in the culture broth, the supernatant can be separated, after completion of the cultivation, from the transformant or cell to collect the supernatant by a publicly known method.

The supernatant or the protein contained in the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the protein thus obtained is in a free form, the protein can be converted into the salt by publicly known methods or modifications thereof. On the other hand, when the protein is obtained in the form of a salt, it can be converted into the free form or in the form of a different salt by publicly known methods or modifications thereof.

The protein produced by the recombinant can be treated, prior to or after the purification, with an appropriate protein-modifying enzyme so that the protein can be appropriately modified to partially remove the polypeptide. Examples of the protein-modifying enzyme include trypsin, chymotrypsin, arginyl endopeptidase, protein kinase, glycosidase and the like.

The presence of the thus produced protein of the present invention can be determined by a binding test to a labeled ligand and by an enzyme immunoassay using a specific antibody.

The antibodies to the protein or partial peptide, or its salts used in the present invention may be any of polyclonal and monoclonal antibodies, as long as they are capable of recognizing the protein or partial peptide, or its salts, used in the present invention.

The antibodies to the protein or partial peptide, or its salts used in the present invention (hereinafter sometimes simply referred to as the protein of the invention in describing the antibodies) may be produced by a publicly known method of producing an antibody or antiserum, using the protein of the invention as an antigen.

**[Preparation of monoclonal antibody]**

**(a) Preparation of monoclonal antibody-producing cells**

The protein of the present invention is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every two to six weeks and twice to ten times in total. Examples of the applicable warm-blooded animals are monkeys, rabbits, dogs, guinea pigs, mice, rats, sheep, goats and chickens, with the use of mice and rats being preferred.

In the preparation of monoclonal antibody-producing cells, a warm-blooded animal, e.g., mice, immunized with an antigen wherein the antibody titer is noted is selected, then spleen or lymph node is collected after two to five days from the final immunization and antibody-producing cells contained therein are fused with myeloma cells from homozoic or heterozoic animal to give monoclonal antibody-producing hybridomas. Measurement of the antibody titer in antisera may be carried out, for example, by reacting a labeled protein, which will be described later, with the antiserum followed by assaying the binding activity of the labeling agent bound to the antibody. The fusion may be carried out, for example, by the known method by Koehler and Milstein [Nature, 256, 495, (1975)]. Examples of the fusion accelerator are polyethylene glycol (PEG), Sendai virus, etc., of which PEG is preferably employed.

Examples of the myeloma cells are those collected from warm-blooded animals such as NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubation at 20 to 40°C, preferably at 30 to 37°C for 1 to 10 minutes, an efficient cell fusion can be carried out.

Various methods can be used for screening of monoclonal antibody-producing hybridomas. Examples of such methods include a method which comprises adding the supernatant of a hybridoma to a solid phase (e.g., a microplate) adsorbed with the protein as an antigen directly or together with a carrier, adding an anti-immunoglobulin antibody (where mouse cells are used for the cell fusion, anti-mouse immunoglobulin antibody is used) labeled with a radioactive substance or an enzyme or Protein A and detecting the monoclonal antibody bound to the solid phase, and a method which comprises adding the supernatant of hybridoma to a solid phase adsorbed with an anti-immunoglobulin antibody or Protein A, adding the protein labeled with a radioactive substance or an enzyme and detecting the monoclonal antibody bound to the solid phase, or the like.

The monoclonal antibody can be screened according to publicly known methods or their modifications. In general, the screening can be effected in a medium for animal cells supplemented with HAT (hypoxanthine, aminopterin and thymidine). Any screening and growth medium can be employed as far as the hybridoma can grow there. For example, RPMI 1640 medium containing 1% to 20%, preferably 10% to 20% fetal bovine serum, GIT medium (Wako Pure Chemical Industries, Ltd.) containing 1 to 10% fetal bovine serum, a serum free medium for cultivation of a hybridoma (SFM-101, Nissui Seiyaku Co., Ltd.) and the like, can be used for the screening and growth medium. The culture is carried out generally at 20°C to 40°C, preferably at 37°C, for about 5 days to about 3 weeks, preferably 1 to 2 weeks, normally in 5% $CO_2$. The antibody titer of the culture supernatant of a hybridoma can be determined as in the assay for the antibody titer in antisera described above.

**(b) Purification of monoclonal antibody**

Separation and purification of a monoclonal antibody can be carried out by publicly known methods, such as separation and purification of immunoglobulins [for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A or Protein G and dissociating the binding to obtain the antibody.]

**[Preparation of polyclonal antibody]**

The polyclonal antibody of the present invention can be manufactured by publicly known methods or modifications thereof. For example, a warm-blooded animal is immunized with an immunogen (protein antigen) per se, or a complex of immunogen and a carrier protein is formed and a warm-blooded animal is immunized with the complex in a manner similar to the method described above for the manufacture of monoclonal antibodies. The product containing the antibody to the protein of the present invention is collected from the immunized animal followed by separation and purification of the antibody.

In the complex of immunogen and carrier protein used to immunize a warm-blooded animal, the type of carrier protein and the mixing ratio of carrier to hapten may be any type and in any ratio, as long as the antibody is efficiently produced to the hapten immunized by crosslinking to the carrier. For example, bovine serum albumin, bovine thyroglobulin or hemocyanin is coupled to hapten in a carrier-to-hapten weight ratio of approximately 0.1 to 20, preferably about 1 to about 5.

A variety of condensation agents can be used for the coupling of carrier to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every 2 to 6 weeks and 3 to 10 times in total.

The polyclonal antibody can be collected from the blood, ascites, etc., preferably from the blood of warm-blooded animal immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as in the separation and purification of monoclonal antibodies described hereinabove.

The antisense nucleotide having a complementary or substantial complementary base sequence to the DNA encoding the protein or partial peptide used in the present invention (hereinafter these DNAs are sometimes collectively referred to as the DNAs of the present invention in the following description of antisense nucleotide) can be any antisense nucleotide, so long as it possesses a base sequence complementary or substantially complementary to the base sequence of the DNA of the present invention and capable of suppressing expression of the DNA, but it is preferably an antisense DNA.

The base sequence substantially complementary to the DNA of the present invention may, for example, be a base sequence having at least about 70% homology, preferably at least about 80% homology, more preferably at least about 90% homology and most preferably at least about 95% homology, to the full-length base sequence or partial base sequence of the base sequence complementary to the DNA of the present invention (i.e., complementary strand to the DNA used in the present invention), and the like. In the entire base sequence of the complementary strand to the DNA of the present invention, an antisense nucleotide having at least about 70% homology, preferably at least about 80% homology, more preferably at least about 90% homology and most preferably at least about 95% homology, to the complementary strand of the base sequence which encodes the N-terminal region in the protein of the present invention (e.g., the base sequence around the initiation codon).

Specifically, there may be enumerated a nucleotide sequence complementary or substantially complementary to the nucleotide sequence of a DNA having the nucleotide sequence represented by SEQ ID NO: 2, or an antisense polynucleotide having a part thereof; preferably, for example, a nucleotide sequence complementary to the nucleotide sequence of a DNA having the nucleotide sequence represented by SEQ ID NO: 2, or an antisense polynucleotide having a part thereof (more preferably, a nucleotide sequence complementary to the nucleotide sequence of a DNA having the nucleotide sequence represented by SEQ ID NO: 2, or an antisense polynucleotide having a part thereof).

The antisense nucleotide is generally constructed by bases of about 10 to about 40, preferably about 15 to about 30.

To prevent digestion with a hydrolase such as nuclease, etc., the phosphoric acid residue (phosphate) of each nucleotide that constitutes the antisense DNA may be substituted with chemically modified phosphoric acid residues, e.g., phosphorothioate, methylphosphonate, phosphorodithionate, etc. These antisense nucleotides may be synthesized using a publicly known DNA synthesizer, etc.

According to the present invention, antisense polynucleotides (nucleic acids) that can inhibit replication or expression of the protein gene of the present invention can be designed and synthesized based on the cloned or determined base sequence information of the DNA encoding the protein. Such polynucleotides (nucleic acids) can hybridize to the RNA of the protein gene and inhibit RNA synthesis or the function of RNA, or can regulate/control the expression of the protein gene via the interaction with RNAs associated with the protein. Polynucleotides complementary to the specified sequences of RNA associated with the protein and polynucleotides that can specifically hybridize to RNA associated with the protein are useful for regulating and controlling the expression of the protein gene in vivo and in vitro. These polynucleotides are also useful for the treatment and diagnosis of diseases. The term "correspond" is used to refer to homologous or complementary to a specific sequence of nucleotides, base sequences or nucleic acids including the gene. As between nucleotides, base sequences or nucleic acids and peptides (proteins), the term "corresponding" usually refers to amino acids of a peptide (protein) that is instructed to be derived from the sequence of nucleotides (nucleic acids) or its complements. The 5' end hairpin loop, 5' end 6-base-pair repeats, 5' end untranslated region, polypeptide translation initiation codon, protein coding region, ORF translation initiation codon, 3' untranslated region, 3' end palindrome region, and 3' end hairpin loop of the protein gene may be selected as preferred target regions, though any region may be a target within the protein genes.

The relationship between the targeted nucleic acids and the polynucleotides complementary to at least a portion of the target, specifically the relationship between the target and the polynucleotides hybridizable to the target, is denoted to be "antisense". The antisense polynucleotides may be polydeoxynucleotides containing 2-deoxy-D-ribose, polydeoxynucleotides containing D-ribose, any other type of polynucleotides which are N-glycosides of a purine or pyrimidine base, or other polymers containing non-nucleotide backbones (e.g., protein nucleic acids and synthetic sequence-specific nucleic acid polymers commercially available) or other polymers containing nonstandard linkages (provided that the polymers contain nucleotides with such a configuration that allows base pairing or base stacking, as is found in DNA and RNA). The antisense polynucleotides may be double-stranded DNA, single-stranded DNA, single-stranded RNA or a DNA:RNA hybrid, and further includes unmodified polynucleotides (or unmodified oligonucleotides), those with publicly known types of modifications, for example, those with labels known in the art, those with caps, methylated polynucleotides, those with substitution of one or more of naturally occurring nucleotides with their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (including nucleases, nuclease inhibitors, toxins, antibodies, signal peptides, poly-L-lysine, etc.) and saccharides (e.g., monosaccharides, etc.), those with intercalators (e.g., acridine, psoralen, etc.), those containing chelators (e.g., metals, radioactive metals, boron, oxidative metals, etc.) and those containing alkylating agents, those with modified linkages (e.g., $\alpha$ anomeric nucleic acids, etc.). Herein the terms "nucleoside", "nucleotide" and "nucleic acid" are used to refer to moieties that contain not only the purine and pyrimidine bases, but also other heterocyclic bases, which have been modified. Such modifications include methylated purines and pyrimidines, acylated purines and pyrimidines and other heterocyclic rings. Modified nucleosides or nucleotides also include modifications on the sugar moiety, for example, wherein one or more hydroxyl groups may optionally be replaced with a halogen, aliphatic groups, or may be converted into the corresponding functional groups such as ethers, amines, or the like.

The antisense polynucleotide (nucleic acid) of the present invention is RNA, DNA or a modified nucleic acid (RNA, DNA). Specific examples of the modified nucleic acid are, but not limited to, sulfurized and thiophosphate derivatives of nucleic acids and those resistant to degradation of polynucleoside or oligonucleoside amides. The antisense nucleic acids of the present invention can be modified preferably based on the following design, that is, by increasing the intracellular stability of the antisense nucleic acid, increasing the cellular permeability of the antisense nucleic acid, increasing the affinity of the nucleic acid to the target sense strand to a higher level, or minimizing the toxicity, if any, of the antisense nucleic acid.

Many such modifications are known in the art, as disclosed in J. Kawakami et al., Pharm. Tech. Japan, Vol. 8, pp.247, 1992; Vol. 8, pp.395, 1992; S. T. Crooke et al. ed., Antisense Research and Applications, CRC Press, 1993; etc.

The antisense nucleic acid of the present invention may contain altered or modified sugars, bases or linkages. The antisense nucleic acid may also be provided in a specialized form such as liposomes, microspheres or may be applied to gene therapy or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached at the 3' or 5' ends of the nucleic acid and may be also attached through a base, sugar, or intramolecular nucleoside linkage. Other moieties may be capping groups specifically placed at the 3' or 5' ends of the nucleic acid to prevent degradation by nuclease such as exonuclease, RNase, etc. Such capping groups include, but are not limited to, hydroxyl protecting groups known in the art, including glycols such as polyethylene glycol, tetraethylene glycol and the like.

The inhibitory activity of the antisense nucleic acid can be examined using the transformant of the present invention, the gene expression system of the present invention in vitro and in vivo, or the translation system of the protein of the present invention in vitro and in vivo. The nucleic acid can be applied to cells by a variety of publicly known methods.

Hereinbelow, uses of the protein or a partial peptide thereof or a salt thereof of the present invention (hereinafter, sometimes simply referred to as the protein of the present invention), the polynucleotide encoding the protein or partial peptide of the present invention (preferably DNA (hereinafter, sometimes simply referred to as the DNA of the present invention)), the antibody to the protein, partial peptide, or salt of the present invention (hereinafter, sometimes simply referred to as the antibody of the present invention) and the antisense polynucleotide to the DNA of the present invention (hereinafter, sometimes simply referred to as the antisense polynucleotide of the present invention) will be explained.

Since the expression of the protein of the present invention is increased tissue-specifically in chronic obstructive pulmonary diseases, the protein may be utilized as a disease marker. Specifically, the protein of the present invention is useful as a marker for making early diagnosis, judging the severity of conditions, and forecasting the progress of diseases in pulmonary/thoracic diseases and respiratory diseases accompanied by lung/airway inflammation [e.g. chronic obstructive pulmonary diseases (such as chronic bronchitis, pulmonary emphysema, diffuse panbronchiolitis, intrinsic asthma), bronchial asthma, cystis fibrosis, hypersensitivity pneumonitis, pulmonary fibrosis, etc.], inflammatory bowel diseases, and allergic conjunctivitis.

Medicines comprising an antisense polynucleotide to a gene encoding the protein of the present invention, a compound or a salt thereof that inhibits the activity of the protein of the present invention, or an antibody to the protein of the present invention may be used as prophylactic and/or therapeutic agents for pulmonary/thoracic diseases and respiratory diseases accompanied by lung/airway inflammation [e.g. chronic obstructive pulmonary diseases (such as chronic bronchitis, pulmonary emphysema, diffuse panbronchiolitis, intrinsic asthma), bronchial asthma, cystis fibrosis, hypersensitivity pneumonitis, pulmonary fibrosis, etc.], inflammatory bowel diseases, allergic conjunctivitis, and so on.

(1) Screening for Candidate Compounds for Medicine against Diseases

Since the expression of the protein of the present invention is increased prior to lung and/or airway inflammation, a compound or salt thereof that inhibits the activity of the protein of the present invention can be used as a medicine (such as prophylactic and/or therapeutic agent) for pulmonary/thoracic diseases and respiratory diseases accompanied by lung/airway inflammation [e.g. chronic obstructive pulmonary diseases (such as chronic bronchitis, pulmonary emphysema, diffuse panbronchiolitis, intrinsic asthma), bronchial asthma, cystis fibrosis, hypersensitivity pneumonitis, pulmonary fibrosis, etc.], inflammatory bowel diseases, and allergic conjunctivitis.

Therefore, the protein of the present invention is useful as a reagent for screening for compounds or salts thereof that inhibit the activity of the protein of the present invention.

The present invention provides a method of screening for compounds or salts thereof that inhibit the activity (e.g., a chloride channel activity) of the protein of the present invention (hereinafter, sometimes simply referred to as the inhibitor), which comprises using the protein of the present invention.

Specifically, the present invention provides, e.g., a method of screening for the inhibitor, which comprises comparing (i) the case where a cell capable of producing the protein of the present invention is activated with a calcium activator and (ii) the case where a mixture of the cell capable of producing the protein of the present invention and a test compound is activated with a calcium activator.

In the screening method described above, e.g., the chloride channel activities of the protein of the present invention in the cases (i) and (ii) are measured and compared.

As the calcium activator, ionomycin, A23187 (calcimycin), or the like may be used.

The test compound may be, for example, a peptide, protein, non-peptidic compound, synthetic compound, fermentation product, cell extract, plant extract, or animal tissue extract. These compounds may be either novel compounds or known compounds.

The mixing of a cell capable of producing the protein of the present invention with a test compound may be performed either before or after the activation of the cell with a calcium activator. Alternatively, a mixture of a test compound and a calcium activator may be added to a cell capable of producing the protein of the present invention.

To perform the screening method described above, a cell capable of producing the protein of the present invention is suspended in a buffer suitable for screening to prepare a sample. Any buffer having a pH of approximately 4 to 10 (desirably a pH of approximately 6 to 8) such as a phosphate buffer, borate buffer, etc. may be used, as long as it does not inhibit the chloride channel activity of the protein of the present invention.

Examples of the cell capable of producing the protein of the present invention include the aforesaid host (transformant) transformed with a vector containing a DNA encoding the protein of the present invention. Preferably, animal cells such as CHO cells, etc. are used as the host. For the screening, a transformant expressing the protein of the present invention on its cell membrane as a result of cultivation according to the aforementioned method is preferably used.

The chloride channel activity of the protein of the present invention can be assayed by the method described in, e.g., Genomics, 54: 200 (1998), or its modification.

For example, when a test compound inhibits the chloride channel activity in case (ii) described above by about 20% or more, preferably 30% or more, more preferably about 50% or more, as compared to case (i) above, the test compound can be selected as a compound or a salt thereof capable of inhibiting the activity of the protein of the present invention.

The polynucleotide encoding the protein of the present invention is useful as a reagent for screening for compounds or salts thereof that promote or inhibit the expression of the gene for the protein of the present invention.

The present invention also provides a method of screening for compounds or salts thereof that inhibit the expression of the gene for the protein of the present invention (hereinafter, sometimes simply referred to as the inhibitor), which comprises using a polynucleotide encoding the protein of the present invention. More specifically, the present invention provides, e.g., a method of screening for the inhibitor, which comprises comparing:

(iii) the case where a cell capable of producing the protein of the present invention is cultured and (iv) the case where a mixture of the cell capable of producing the protein of the present invention and a test compound is cultured.

In the above-described screening method, e.g., the expression levels of the protein gene of the present invention (specifically, the amounts of the protein of the present invention, or the amounts of mRNA encoding the protein) in cases (iii) and (iv) are measured and compared.

The test compound may be, for example, a peptide, protein, non-peptidic compound, synthetic compound, fermentation product, cell extract, plant extract, or animal tissue extract. These compounds may be either novel compounds or known compounds.

To perform the screening method described above, a cell capable of producing the protein of the present invention is suspended in a buffer suitable for screening to prepare a sample. Any buffer having a pH of approximately 4 to 10 (desirably a pH of approximately 6 to 8) such as a phosphate buffer, borate buffer, etc. may be used, as long as it does not inhibit the chloride channel activity of the protein of the present invention.

Examples of the cell capable of producing the protein of the present invention include the aforesaid host (transformant) transformed with a vector containing a DNA encoding the protein of the present invention. Preferably, animal cells such as CHO cells, etc. are used as the host. For the screening, a transformant expressing the protein of the present invention on its cell membrane as a result of cultivation according to the aforementioned method is preferably used.

The amount of the protein of the present invention may be measured by conventional methods. For example, a method may be used in which the protein present in cell extract or the like is measured using an antibody that recognizes the protein by Western analysis, ELISA, or modifications thereof.

The expression level of the protein gene of the present invention may be measured by conventional methods, e.g., Northern blotting, RT-PCR, real time PCR analysis system (ABI; TaqMan Polymerase Chain Reaction) or modifications thereof.

For example, a test compound that inhibits the expression level of the protein gene of the invention in case (iv) described above by about 20% or more, preferably 30% or more, more preferably about 50% or more, as compared to case (iii) above, can be selected as a compound or a salt thereof capable of inhibiting the expression of the protein gene of the present invention.

Further, the antibody of the invention is useful as a reagent for screening for compounds or salts thereof that inhibit the production (expression) of the protein of the present invention.

The present invention provides a method of screening for compounds or salts thereof that inhibit the production of the protein of the present invention (hereinafter, sometimes simply referred to as the inhibitor), which comprises using antibody of the invention. More specifically, for example, the present invention provides a screening method for the inhibitor, comprising comparing:

(v) the case where a cell capable of producing the protein of the present invention is cultured and (vi) the case where a mixture of the cell capable of producing the protein of the present invention and a test compound is cultured.

In the above-described screening method, e.g., the expression levels of the protein of the present invention (specifically, the amounts of the protein of the present invention) in cases (v) and (vi) are measured using the antibody of the present invention (e.g. detection of the expression of the protein, quantitative determination of the expression level of the protein, etc.), and then compared.

The test compound may be, for example, a peptide, protein, non-peptidic compound, synthetic compound, fermentation product, cell extract, plant extract, or animal tissue extract. These compounds may be either novel compounds or known compounds.

To perform the screening method described above, a cell capable of producing the protein of the present invention is suspended in a buffer suitable for screening to prepare a sample. Any buffer having a pH of approximately 4 to 10 (desirably a pH of approximately 6 to 8) such as a phosphate buffer, borate buffer, etc. may be used, as long as it does not inhibit the chloride channel activity of the protein of the present invention.

Examples of the cell capable of producing the protein of the present invention include the aforesaid host (transformant) transformed with a vector containing a DNA encoding the protein of the present invention. Preferably, animal cells such as CHO cells, etc. are used as the host. For the screening, a transformant expressing the protein of the present invention on its cell membrane as a result of cultivation according to the aforementioned method is preferably used.

The amount of the protein of the present invention may be measured by conventional methods. For example, a method may be used in which the protein present in cell extract or the like is measured using an antibody that recognizes the protein by Western analysis, ELISA, or modifications thereof.

For example, a test compound that inhibits the expression level of the protein of the present invention in case (vi) above by about 20% or more, preferably 30% or more, more preferably about 50% or more, as compared to case (v) above, can be selected as a compound or a salt thereof capable of inhibiting the expression of the protein of the present invention.

The screening kit of the present invention contains the protein, a partial peptide thereof, or a salt thereof used in the invention, a cell capable of producing the protein or partial peptide thereof used in the invention, a polynucleotide encoding the protein, partial peptide thereof, or salt thereof used in the invention, or the like.

Compounds or salts thereof obtainable by using the screening method or screening kit of the present invention are compounds or salts thereof selected from the above-described test compounds, such as peptides, proteins, non-peptidic compounds, synthetic compounds, fermentation products, cell extracts, plant extracts, animal tissue extracts or plasma; and are compounds or salts thereof that inhibit the activity (e.g. chloride channel activity, etc.) of the protein of the present invention, compounds or salts thereof that inhibit the expression of the gene for the protein of the present invention, or compounds or salts thereof that inhibit the production of the protein of the present invention.

As salts of such compounds, those salts described earlier on the salts of the protein of the present invention may be used.

The compound or salt thereof that inhibits the activity of the protein of the present invention, the compound or salt thereof that inhibits the expression of the gene for the protein of the present invention, or the compound or salt thereof that inhibits the production of the protein of the present invention is useful as a medicine, such as a prophylactic and/or therapeutic agent, for pulmonary/thoracic diseases and respiratory diseases accompanied by pulmonary/airway inflammation [e.g. chronic obstructive pulmonary diseases (such as chronic bronchitis, pulmonary emphysema, diffuse panbronchiolitis, intrinsic asthma), bronchial asthma, cystis fibrosis, hypersensitivity pneumonitis, pulmonary fibrosis, etc.], inflammatory bowel diseases, allergic conjunctivitis, and so on.

When the compound or salt thereof described above is used as the prophylactic and/or therapeutic agent described above, the compound or salt may be formulated into preparations, such as tablets, capsules, elixirs, microcapsules, aseptic solutions, or suspensions, according to conventional means. For example, the compound or salt may be used orally in the form of tablets (sugar-coated, if necessary), capsules, elixirs, microcapsules or the like; or parenterally in the form of injections such as aseptic solutions or suspensions in water or other pharmaceutically acceptable liquids.

These preparations may be produced, for example, by mixing the compound or salt thereof with physiologically acceptable carriers, flavoring agents, excipients, vehicles, antiseptics, stabilizers, binders, etc. in unit dosage forms required for preparing generally approved pharmaceutical preparations. The amounts of active ingredients in these formulations are decided so that an appropriate dose within the specified range can be obtained.

Additives miscible with tablets, capsules etc. include a binder such as gelation, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated according to a conventional manner used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition.

Examples of an aqueous medium for injection include physiological saline and an isotonic solution containing glucose and other auxiliary agents (e.g., D-sorbitol, D-mannitol and sodium chloride) and may be used in combination with an appropriate dissolution aid such as an alcohol (e.g., ethanol), a polyalcohol (e.g., propylene glycol and polyethylene glycol), a nonionic surfactant (e.g., polysorbate 80™ and HCO-50), etc. As an oily medium, for example, sesame oil and soybean oil may be used, which can be used in combination with a dissolution aid such as benzyl benzoate and benzyl alcohol. Furthermore, a buffer (e.g., phosphate buffer and sodium acetate buffer), a soothing agent (e.g., benzalkonium chloride, procaine hydrochloride), a stabilizer (e.g., human serum albumin, polyethylene glycol), a preservative (e.g., benzyl alcohol, phenol), an antioxidant, etc. may be added. The thus-prepared liquid for injection is normally filled in an appropriate ampoule.

Since the thus obtained pharmaceutical preparation is safe and low toxic, the preparation can be administered to human or other warm-blooded animal (e.g., mouse, rat, rabbit, sheep, swine, bovine, horse, chicken, cat, dog, monkey, chimpanzee, etc.).

The dose of the compound or salt thereof varies depending on its action, target disease, subject to be administered, route for administration, etc.; when the compound or its salt that inhibits the activities of the protein of the present invention is orally administered for the treatment of, e.g., bronchial asthma, the compound or its salt is normally administered in a dose of about 0.1 to about 100 mg, preferably about 1.0 to about 50 mg, and more preferably about 1.0 to about 20 mg per day for adult (as 60 kg body weight). In parenteral administration, a single dose of the compound or its salt varies depending on subject to be administered, target disease, etc. but it is advantageous for the treatment of, e.g., bronchial asthma, to administer the compound or its salt capable of inhibiting the activities of the protein of the present invention intravenously in the form of injection in a daily dose of about 0.01 to about 30 mg, preferably about 0.1 to about 20 mg, and more preferably about 0.1 to about 10 mg for adult (as 60 kg body weight). For other animal species, the corresponding dose as converted per 60 kg body weight can be administered.

**(2) Quantification for the protein of the invention or its peptide, or salts thereof**

The antibody to the protein of the present invention (hereinafter sometimes merely referred to as the antibody of the present invention) is capable of specifically recognizing the protein of the present invention, and can thus be used for a quantification of the protein of the present invention in a test sample fluid, in particular, for a quantification by sandwich immunoassay, etc.

That is, the present invention provides:

(i) a method for quantification of the protein of the present invention in a test sample fluid, which comprises competitively reacting the antibody of the present invention, a test sample fluid and the labeled protein of the present invention, and measuring the ratio of the labeled protein of the present invention bound to said antibody; and,

(ii) a method for quantification of the protein of the present invention in a test sample fluid, which comprises reacting the test sample fluid simultaneously or continuously with the antibody of the present invention immobilized on a carrier and another labeled antibody of the present invention, and then measuring the activity of the labeling agent on the immobilized carrier.

In the method (ii) for quantification described above, it is preferred that one antibody is capable of recognizing the N-terminal region of the protein of the present invention, while another antibody is capable of recognizing the C-terminal region of the protein of the present invention.

The monoclonal antibody to the protein of the present invention (hereinafter sometimes referred to as the monoclonal antibody of the invention) may be used to assay the protein of the present invention. Furthermore, the protein can be detected by means of a tissue staining as well. For these purposes, the antibody molecule per se may be used or $F(ab')_2$, Fab' or Fab fractions of the antibody molecule may also be used.

There is no particular limitation to the method for quantification of the protein of the present invention using the antibody of the present invention; any method may be used, so far as it relates to a method, in which the amount of an antibody, antigen or antibody-antigen complex can be detected by a chemical or physical means, depending on or corresponding to the amount of antigen (e.g., the amount of a protein) in a test sample fluid to be assayed, and then calculated using a standard curve prepared by a standard solution containing the known amount of antigen. Advantageously used are, for example, nephrometry, competitive method, immunometric method and sandwich method; in terms of sensitivity and specificity, the sandwich method, which will be described later, is particularly preferred.

Examples of the labeling agent used in the assay method using the labeling substance are radioisotopes, enzymes, fluorescent substances, luminescent substances, and the like. Examples of the radioisotope are [$^{125}I$], [$^{131}I$], [$^{3}H$], [$^{14}C$], etc. Preferred examples of the enzyme described above are those that are stable and have a high specific activity, which include β-galactosidase, β-glucosidase, alkaline phosphatase, peroxidase, malate dehydrogenase, etc. Examples of the fluorescent substance are fluorescamine, fluorescein isothiocyanate, etc. Examples of the luminescent substance are luminol, a luminol derivative, luciferin, lucigenin, etc. Furthermore, the biotin-avidin system may also be used for binding of an antibody or antigen to a labeling agent.

In the immobilization of antigens or antibodies, physical adsorption may be used. Alternatively, chemical binding that is conventionally used for immobilization of proteins or enzymes may be used as well. Examples of the carrier include insoluble polysaccharides such as agarose, dextran and cellulose; synthetic resins such as polystyrene, polyacrylamide and silicone; glass; etc.

In the sandwich method, a test sample fluid is reacted with an immobilized monoclonal antibody of the present invention (first reaction), then reacted with another labeled monoclonal antibody of the present invention (second reaction) and the activity of the labeling agent on the immobilized carrier is assayed, whereby the amount of the protein of the present invention in the test sample fluid can be quantified. The first and second reactions may be carried out in a reversed order, simultaneously or sequentially with an interval. The type of the labeling agent and the method for immobilization may be the same as those described hereinabove. In the immunoassay by the sandwich method, it is not always necessary that the antibody used for the labeled antibody and for the solid phase should be one type or one species but a mixture of two or more antibodies may also be used for the purpose of improving the measurement sensitivity, etc.

In the method according to the present invention for assaying the protein of the present invention by the sandwich method, preferred monoclonal antibodies of the present invention used for the first and the second reactions are antibodies, which binding sites to the protein of the present invention are different from one another. That is, the antibodies used in the first and the second reactions are those wherein, when the antibody used in the second reaction recognizes the C-terminal region of the protein of the present invention, the antibody recognizing the site other than the C-terminal regions, e.g., recognizing the N-terminal region, is preferably used in the first reaction.

The monoclonal antibody of the present invention may be used in an assay system other than the sandwich method, such as a competitive method, an immunometric method, a nephrometry, etc.

In the competitive method, an antigen in a test sample fluid and a labeled antigen are competitively reacted with an antibody, then the unreacted labeled antigen (F) and the labeled antigen bound to the antibody (B) are separated (i.e., B/F separation) and the labeled amount of either B or F is measured to determine the amount of the antigen in the test sample fluid. In the reactions for such a method, there are a liquid phase method in which a soluble antibody is used as the antibody and the B/F separation is effected by polyethylene glycol while a second antibody to the antibody is used, and a solid phase method in which an immobilized antibody is used as the first antibody or a soluble antibody is used as the first antibody while an immobilized antibody is used as the second antibody.

In the immunometric method, an antigen in a test sample fluid and an immobilized antigen are competitively reacted with a given amount of a labeled antibody followed by separating the solid phase from the liquid phase; or an antigen in a test sample fluid and an excess amount of labeled antibody are reacted, then an immobilized antigen is added to bind an unreacted labeled antibody to the solid phase and the solid phase is separated from the liquid phase. Thereafter, the labeled amount of any of the phases is measured to determine the antigen amount in the test sample fluid.

In the nephrometry, the amount of insoluble sediment, which is produced as a result of the antigen-antibody reaction in a gel or in a solution, is measured. Even when the amount of an antigen in a test sample fluid is small and only a small amount of the sediment is obtained, a laser nephrometry utilizing laser scattering can be suitably used.

In applying each of those immunoassays to the assay method of the present invention, any special conditions or operations are not required to set forth. The assay system for the protein of the present invention may be constructed in addition to conditions or operations conventionally used for each of the methods, taking the technical consideration of one skilled in the art into account consideration. For the details of such conventional technical means, a variety of reviews, reference books, etc. may be referred to.

For example, there are Hiroshi Irie (ed.): "Radioimmunoassay" (published by Kodansha, 1974); Hiroshi Irie (ed.): "Radioimmunoassay; Second Series" (published by Kodansha, 1979); Eiji Ishikawa, et al. (ed.): "Enzyme Immunoassay" (published by Igaku Shoin, 1978); Eiji Ishikawa, et al. (ed.): "Enzyme Immunoassay" (Second Edition) (published by Igaku Shoin, 1982); Eiji Ishikawa, et al. (ed.): "Enzyme Immunoassay" (Third Edition) (published by Igaku Shoin, 1987); "Methods in Enzymology" Vol. 70 (Immunochemical Techniques (Part A)); ibid., Vol. 73 (Immunochemical Techniques (Part B)); ibid., Vol. 74 (Immunochemical Techniques (Part C)); ibid., Vol. 84 (Immunochemical Techniques (Part D: Selected Immunoassays)); ibid., Vol. 92 (Immunochemical Techniques (Part E: Monoclonal Antibodies and General Immunoassay Methods)); ibid., Vol. 121 (Immunochemical Techniques (Part I: Hybridoma Technology and Monoclonal Antibodies)) (published by Academic Press); etc.)

As described above, the protein of the present invention can be quantified with high sensitivity, using the antibody of the present invention.

Furthermore, (1) when an increased level of the protein of the present invention is detected by quantifying the level of the protein of the present invention using the antibody of the present invention, it can be diagnosed that one suffers from diseases such as pulmonary/thoracic diseases and respiratory diseases accompanied by pulmonary/airway inflammation [e.g. chronic obstructive pulmonary diseases (such as chronic bronchitis, pulmonary emphysema, diffuse panbronchiolitis, intrinsic asthma), bronchial asthma, cystis fibrosis, hypersensitivity pneumonitis, pulmonary fibrosis, etc.], inflammatory bowel diseases, allergic conjunctivitis, etc., or it is highly likely to suffer from these disease in the future.

The antibody of the present invention can be employed for detecting the protein of the present invention, which is present in a test sample fluid such as a body fluid, a tissue, etc. The antibody can also be used to prepare an antibody column for purification of the protein of the present invention, detect the protein of the present invention in each fraction upon purification, and analyze the behavior of the protein of the present invention in the cells under investigation.

**(3) Gene diagnostic agent**

By using the polynucleotide (such as DNA) of the present invention, e.g., as a probe, an abnormality (gene abnormality) of the DNA or mRNA encoding the protein of the present invention or its partial peptide in human or warm-blooded animal (e.g., rat, mouse, guinea pig, rabbit, chicken, sheep, swine, bovine, horse, cat, dog, monkey, chimpanzee, etc.)can be detected. Therefore, the DNA is useful as a gene diagnostic agent for detecting damages to the DNA or mRNA, its mutation, or decreased expression, increased expression, overexpression, etc. of the DNA or mRNA.

The gene diagnosis described above using the DNA of the present invention can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)), etc.

When over-expression is detected by Northern hybridization or DNA mutation is detected by PCR-SSCP, for example, it may be judged that the relevant subject is very likely to have one of the following diseases: pulmonary/thoracic diseases and respiratory diseases accompanied by pulmonary/airway inflammation [e.g. chronic obstructive pulmonary diseases (such as chronic bronchitis, pulmonary emphysema, diffuse panbronchiolitis, intrinsic asthma), bronchial asthma, cystis fibrosis, hypersensitivity pneumonitis, pulmonary fibrosis, etc.], inflammatory bowel diseases, allergic conjunctivitis and so on.

**(4) Medicine and Diagnostic Agents Comprising Antisense Polynucleotide**

The antisense polynucleotide of the present invention capable of complementarily binding to the DNA of the present invention to thereby inhibit the expression of the DNA is of low toxicity and can inhibit the *in vivo* function (e.g. chloride channel activity) of the protein or the DNA or the present invention. Therefore, the polynucleotide of the present invention may be used as a prophylactic and/or therapeutic agent for pulmonary/thoracic diseases and respiratory diseases accompanied by pulmonary/airway inflammation [e.g. chronic obstructive pulmonary diseases (such as chronic bronchitis, pulmonary emphysema, diffuse panbronchiolitis, intrinsic asthma), bronchial asthma, cystis fibrosis, hypersensitivity pneumonitis, pulmonary fibrosis, etc.], inflammatory bowel diseases, allergic conjunctivitis and so on.In the case that the antisense nucleotide described above is used as the therapeutic/prophylactic agent described above, the antisense nucleotide can be prepared into pharmaceutical preparations and administered by a publicly known method.

For example, when the antisense nucleotide is used, the antisense nucleotide is administered directly, or the antisense nucleotide is inserted into an appropriate vector such as retrovirus vector, adenovirus vector, adenovirus-associated virus vector, etc., followed by treating in a conventional manner. The antisense nucleotide may be administered as it stands, or with a physiologically acceptable carrier to assist its uptake by gene gun or through a catheter such as a catheter with a hydrogel. Alternatively, the antisense nucleotide may be prepared into an aerosol, which is locally administered intrabronchially as an inhalant.

The dose of the antisense nucleotide varies depending on target disease, subject to be administered, route for administration, etc.; for example, when the antisense nucleotide of the present invention is locally administered intrabronchially as an inhalant for the treatment of, e.g., bronchial asthma, the dose is normally about 0.1 to about 100 mg per day for adult (as 60 kg body weight).

In addition, the antisense nucleotide may also be employed as an oligonucleotide probe for diagnosis to examine the presence of the DNA of the present invention in tissues or cells and states of its expression.

### (5) Medicine or Diagnostic Agents Comprising the Antibody of the Invention

The antibody of the present invention that neutralizes the activity of the protein of the present invention may be used as a medicine for pulmonary/thoracic diseases and respiratory diseases accompanied by pulmonary/airway inflammation [e.g. chronic obstructive pulmonary diseases (such as chronic bronchitis, pulmonary emphysema, diffuse panbronchiolitis, intrinsic asthma), bronchial asthma, cystis fibrosis, hypersensitivity pneumonitis, pulmonary fibrosis, etc.], inflammatory bowel diseases, allergic conjunctivitis and so on.

The aforesaid therapeutic/prophylactic agent containing the antibody of the invention for the diseases described above is low toxic and can be administered orally or parenterally to human or other warm-blooded animal (e.g., rat, rabbit, sheep, swine, bovine, cat, dog, monkey, etc.), in its liquid form as it stands, or as a pharmaceutical composition in a suitable preparation form. The dose varies depending on subject to be administered, target disease, condition, route for administration, etc.; when the pharmaceutical is administered to adult for the treatment/prevention of, e.g., bronchial asthma, the antibody of the present invention is normally advantageously administered intravenously, about 1 to about 5 times a day, preferably about 1 to 3 times a day, in a single dose of about 0.01 to about 20 mg/kg body weight, preferably about 0.1 to about 10 mg/kg body weight, and more preferably about 0.1 to about 5 mg/kg body weight for adult. For other parenteral administration and oral administration, the dose corresponding to the dose above can be administered; when the condition is especially severe, the dose may be increased accordingly to the condition.

The antibody of the present invention may be administered in itself or as an appropriate pharmaceutical composition. The pharmaceutical composition used for the administration described above contains a pharmacologically acceptable carrier with the aforesaid compounds or salts thereof, a diluent or excipient. Such a composition is provided in the preparation suitable for oral or parenteral administration.

That is, examples of the composition for oral administration include solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or an excipient conventionally used in the field of pharmaceutical preparations. Examples of the vehicle or excipient for tablets are lactose, starch, sucrose, magnesium stearate, etc.

Examples of the composition for parenteral administration that can be used are injections, suppositories, etc. and the injections include the form of intravenous, subcutaneous, transcutaneous, intramuscular and drip injections, etc. Such injections are prepared by publicly known methods, e.g., by dissolving, suspending or emulsifying the aforesaid antibody or its salts in a sterile aqueous or oily liquid medium. For the aqueous medium for injection, for example, physiological saline and isotonic solutions containing glucose and other adjuvant, etc. are used. Appropriate dissolution aids, for example, alcohol (e.g., ethanol), polyalcohol (e.g., propylene glycol, polyethylene glycol), nonionic surfactant (e.g., polysorbate 80™, HCO-50 (polyoxyethylene (50 mol) adduct of hydrogenated castor oil)) may be used in combination. For the oily solution, for example, sesame oil, soybean oil and the like are used, and dissolution aids such as benzyl benzoate and benzyl alcohol may be used in combination. The thus-prepared liquid for injection is normally filled in an appropriate ampoule. The suppository used for rectal administration is prepared by mixing the aforesaid antibody or its salts with conventional suppository base.

The oral or parenteral pharmaceutical composition described above is advantageously prepared in a unit dosage form suitable for the dose of the active ingredient. Examples of such unit dosage form include tablets, pills, capsules, injections (ampoules), suppositories, etc. It is preferred that the antibody described above is contained generally in a dose of 5 to 500 mg per unit dosage form, 5 to 100 mg especially for injections and 10 to 250 mg for other dosage forms.

Each composition described above may further contain other active components unless formulation with the antibody causes any adverse interaction.

Further, the antibody of the present invention is also useful as a diagnostic agent for pulmonary/thoracic diseases and respiratory diseases accompanied by pulmonary/airway inflammation [e.g. chronic obstructive pulmonary diseases (such as chronic bronchitis, pulmonary emphysema, diffuse panbronchiolitis, intrinsic asthma), bronchial asthma, cystis fibrosis, hypersensitivity pneumonitis, pulmonary fibrosis, etc.], inflammatory bowel diseases, allergic conjunctivitis and so on.

**(6) Medicines Comprising a Cmpound or a Salt thereof that Inhibits the Activity of the Protein of the Present Invention, a Compound or a Salt thereof that Inhibits the Expression of the Gene for the Protein of the Present Invention, or a Compound or a Salt thereof that Inhibits the Production of the Protein of the Present Invention**

The compound or salt thereof that inhibits the activity of the protein of the present invention, the compound or salt thereof that inhibits the expression of the gene for the protein of the present invention, or the compound or salt thereof that inhibits the production of the protein of the present invention is useful as a medicine, such as a prophylactic and/or therapeutic agent, for pulmonary/thoracic diseases and respiratory diseases accompanied by pulmonary/airway inflammation [e.g. chronic obstructive pulmonary diseases (such as chronic bronchitis, pulmonary emphysema, diffuse panbronchiolitis, intrinsic asthma), bronchial asthma, cystis fibrosis, hypersensitivity pneumonitis, pulmonary fibrosis, etc.], inflammatory bowel diseases, allergic conjunctivitis, and so on.

When the compounds or salts thereof described above are used as the prophylactic and/or therapeutic agent described above, they may be formulated into preparations according to conventional means.In the specification and drawings, the codes of bases and amino acids are denoted in accordance with the IUPAC-IUB Commission on Biochemical Nomenclature or by the common codes in the art, examples of which are shown below. For amino acids that may have the optical isomer, L form is presented unless otherwise indicated.

| | |
|---|---|
| DNA : | deoxyribonucleic acid |
| cDNA : | complementary deoxyribonucleic acid |
| A : | adenine |
| T : | thymine |
| G : | guanine |
| C : | cytosine |
| RNA : | ribonucleic acid |
| mRNA : | messenger ribonucleic acid |
| dATP : | deoxyadenosine triphosphate |
| dTTP : | deoxythymidine triphosphate |
| dGTP : | deoxyguanosine triphosphate |
| dCTP : | deoxycytidine triphosphate |
| ATP : | adenosine triphosphate |
| EDTA : | ethylenediaminetetraacetic acid |
| SDS : | sodium dodecyl sulfate |
| Gly : | glycine |
| Ala : | alanine |
| Val : | valine |
| Leu : | leucine |
| Ile : | isoleucine |
| Ser : | serine |
| Thr : | threonine |
| Cys : | cysteine |
| Met : | methionine |
| Glu : | glutamic acid |
| Asp : | aspartic acid |
| Lys : | lysine |
| Arg : | arginine |
| His : | histidine |
| Phe : | phenylalanine |
| Tyr : | tyrosine |
| Trp : | tryptophan |
| Pro : | proline |

Asn : asparagine
Gln : glutamine
pGlu : pyroglutamic acid

Substituents, protecting groups, and reagents used in this specification are presented as the codes below.

Me : methyl group
Et : ethyl group
Bu : butyl group
Ph : phenyl group
TC : thiazolidine-4(R)-carboxamide group
Tos : p-toluenesulfonyl
CHO : formyl
Bzl : benzyl
$Cl_2$-Bzl : : 2,6-dichlorobenzyl
Bom : benzyloxymethyl
Z : benzyloxycarbonyl
Cl-Z : 2-chlorobenzyl oxycarbonyl
Br-Z : 2-bromobenzyl oxycarbonyl
Boc : t-butoxycarbonyl
DNP : dinitrophenol
Trt : trityl
Bum : t-butoxymethyl
Fmoc : N-9-fluorenyl methoxycarbonyl
HOBt : 1 -hydroxybenztriazole
HOOBt : 3,4-dihydro-3-hydroxy-4-oxo-1,2,3benzotriazine
HONB : 1-hydroxy-5-norbornene-2,3-dicarboxyimide
DCC : N,N'-dichlorohexylcarbodiimide

The sequence identification numbers in the sequence listing of the specification indicate the following sequences.

[SEQ ID NO:1]

This shows the amino acid sequence of human CLCA4 protein.

[SEQ ID NO: 2]

This shows the nucleotide sequence of a DNA encoding human CLCA4 protein having the amino acid sequence represented by SEQ ID NO: 1.

[SEQ ID NO: 3]

This shows the nucleotide sequence of primer 1 used in Example 1.

[SEQ ID NO: 4]

This shows the nucleotide sequence of primer 2 used in Example 1.

[SEQ ID NO: 5]

This shows the nucleotide sequence of the TaqMan probe used in Example 1.

[SEQ ID NO: 6]

This shows the nucleotide sequence of the DNA obtained in Example 2.

[SEQ ID NO: 7]

This shows the nucleotide sequence of a primer used in Example 2.

[SEQ ID NO: 8]

This shows the nucleotide sequence of a primer used in Example 2.

[SEQ ID NO: 9]

This shows the nucleotide sequence of a primer used in Example 2.

[SEQ ID NO: 10]

This shows the nucleotide sequence of a primer used in Example 2.

[SEQ ID NO: 11]

This shows the nucleotide sequence of a primer used in Example 2.

[SEQ ID NO: 12]

This shows the nucleotide sequence of a primer used in Example 2.

[SEQ ID NO: 13]

This shows the nucleotide sequence of a primer used in Example 2.

[SEQ ID NO: 14]

This shows the nucleotide sequence of a primer used in Example 2.

[SEQ ID NO: 15]

This shows the nucleotide sequence of a primer used in Example 2.

[SEQ ID NO: 16]

This shows the nucleotide sequence of a primer used in Example 2.

[SEQ ID NO: 17]

This shows the nucleotide sequence of a primer used in Example 2.

[SEQ ID NO: 18]

This shows the nucleotide sequence of a primer used in Example 2.

[SEQ ID NO: 19]

This shows the nucleotide sequence of a primer used in Example 2.

[SEQ ID NO: 20]

This shows the nucleotide sequence of a primer used in Example 2.

## EXAMPLES

Hereinafter the present invention will be described in more detail with reference to EXAMPLES and REFERENCE EXAMPLES, but is not deemed to be limited thereto. The gene manipulation procedures using *Escherichia coli* were performed according to the methods described in the Molecular Cloning.

## EXAMPLE 1

### Expression of CLCL4 Gene Family in Chronic Obstructive Pulmonary Disease (COPD) Patients

Induced sputum samples were taken from COPD patients (n=14) and non-smoking healthy individuals (n=12). Total RNA was extracted from each induced sputum sample using ISOGEN (Wako Purechemical Industries). Using 100 ng of the total RNA as a starting material, cDNA was synthesized in 100 µl of a reaction solution by reverse transcription reaction using TaqMan Gold RT-PCR Kit (Applied Biosystems). Then, using 10 µl of the reaction solution, the number of copies of CLCA4 gene was determined by TaqMan PCR using ABI PRISM 7700 sequence detector (Applied Biosystems). The primers [primer 1 (SEQ ID NO: 3); primer 2 (SEQ ID NO: 4)] and TaqMan probe (SEQ ID NO: 5) used for the detection of quantities of the gene were designed using Primer Express Program. As a reporter dye for the TaqMan probe, 6-carboxyfluorescein (FAM) was used. In order to eliminate non-specific amplification, samples without reverse transcriptase were also treated in the same manner, and the number of gene copies per total RNA was determined with the formula described below. Then, the expression of the gene was compared between COPD patients and non-smoking healthy individuals.

(Number of gene copies in sample with reverse transcriptase) - (Number of gene

copies in sample without reverse transcriptase) = (Number of gene copies)

The results are shown in Fig. 1.
From these results, it was found that the expression of CLCA4 gene (SEQ ID NO: 4) was increased significantly in COPD patients (**$p<0.01$).

## EXAMPLE 2

### (1) Acquisition of Human CLCA4 Gene

Total RNA was prepared from primary cultures of human bronchial epithelial cells (NHBE cells; Takara Shuzo) using ISOGEN (Wako Purechemical Industries). Using 200 ng of the total RNA as a starting material, cDNA was synthesized by reverse transcription reaction using Marathon cDNA Amplification kit (Clontech). Using this cDNA as a template, a PCR was performed with two primer DNAs: CLCA4 gene 5'-untranslated region primer [primer 3 (SEQ ID NO: 7)] and 3'-untraanslated region primer [primer 4 (SEQ ID NO: 8)]. As a result, full-length CLCA4 gene was obtained (SEQ ID NO: 6). The PCR reaction was performed as follows in a thermal cycler Gene Amp PCR System 9700 (Perkin Elmer) using Takara Pyrobest (Takara Shjuzo): at 94°C for 1 min, then 35 cycles of at 94°C for 10 sec, at 60°C for 30 sec and at 72°C for 4 min, and finally at 72°C for 10 min. The resultant DNA fragment of full-length CLCA4 gene was cloned into pCRII-Blunt II-TOPO Vector (Invitrogen). Further, cycle sequencing reactions were preformed using M13RV primer [primer 5 (SEQ ID NO: 9)], T7 primer [primer 6 (SEQ ID NO: 10)] and synthetic primers [primer 7 (SEQ ID NO: 11), primer 8 (SEQ ID NO: 12), primer 9 (SEQ ID NO: 13), primer 10 (SEQ ID NO: 14), primer 11 (SEQ ID NO: 15), primer 12 (SEQ ID NO: 16), primer 13 (SEQ ID NO: 17), primer 14 (SEQ ID NO: 18), primer 15 (SEQ ID NO: 19) and primer 16 (SEQ ID NO: 20)], followed by confirmation of the nucleotide sequences of resultant products with fluorescence DNA sequencer (ABI PRISM TM377, Perkin Elmer).

### (2) Construction of a Vector for Expressing CLCA4 Gene in Animal Cells

The CLCA4 gene-inserted pCRII-Blunt II-TOPO Vector mentioned in (1) above was digested with KpnI-NotI. The resultant 2.9 kbp DNA fragment comprising CLCA4 gene was inserted into KpnI-NotI-digested pcDNA3.1 plasmid (Invitrogen) to thereby construct plasmid pcDNA-hCLCA4 which has human CLCA4 gene downstream of cytomegalovirus enhancer/promoter and also has a neomycin resistance gene as a selection marker.

### (3) $Ca^{2+}$ Dependent $Cl^-$ Channel Activity in Human CLCA4-Expressing CHO Cells

The ClCA4 expression plasmid pcDNA-hCLCA4 described in (2) above was introduced into CHO cells using FuGENE 6 (Roche Diagnostics) according to the manual accompanying it. The cells were subjected to selective culture in 1 mg/ml neomycin (G418) (Gibco BRL)-added MEM-α medium (Gibco BRL) for 10 days, and cells growing in this medium were obtained as CLCA4 expressing CHO cells. These CLCA4-expressing CHO cells and parent strain CHO cells were plated in 96-well black clear bottom plates (Coming) at $4x10^4$ cells/well and cultured overnight. $Cl^-$ channel activity was measured using FLIPR Membrane Potential Assay Kit (Molecular Device). A potential sensitive dye (Component A) diluted with 20 mM HEPES-containing HBSS buffer (Component B) was added to the plates at 100 µl/well and reacted at 37°C for 30 min. Then, the plates were set in FLIPR (Molecular Device) and, after addition of ionomycin (Wako Purechemical industries) at a final concentration of 2 µM, changes in fluorescence were observed as $Cl^-$ channel activity. In both parent strain CHO cells and human CLCA4-expressing CHO cells, increase in fluorescence intensity was hardly recognized in the absence of stimulation. When stimulated with 2 µM ionomycin, increase in fluorescence intensity was observed only in human CLCA4-expressing CHO cells (Fig. 2). These results show that CLCA4 functions as $Ca^{2+}$ dependent $Cl^-$ channel.

In the above experimental procedures, when niflumic acid (NFA; Cayman Chemical), an anion transport inhibitor, was added and then cells were left for 10 min at room temperature, the increase in fluorescence intensity caused by stimulation with ionomycin was inhibited in human CLCA4-expressing CHO cells (Fig. 3).

These results revealed that it is possible to screen for compounds or salts thereof that inhibit $Cl^-$ channel using these human CLCA4-expressing CHO cells.

### (4) Screening Using $Ca^{2+}$ Dependent $Cl^-$ Channel Activity as an Indicator

Screening using $Ca^{2+}$ dependent $Cl^-$ channel activity as an indicator is performed with FLIPR Membrane Potential Assay Kit mentioned in (3) above. Briefly, CLCA4-expressing CHO cells are plated in 96-well black clear bottom plates at $4x10^4$ cells/well and cultured overnight. A potential sensitive dye (Component A) diluted with 20 mM HEPES-containing HBSS buffer (Component B) is added to the plates at 75 µl/well and reacted at 37°C for 20 min. Then, Component B or a test compound diluted to a specific concentration with Component B is added to these plates at 25 µl/well and reacted at 37°C for 10 min. Subsequently, the plates are set in FLIPR (Molecular Device), and Component B or Component B with ionomycin at a final concentration of 2 µM is added thereto at 50 µl/well. Changes in fluorescence after the addition are observed as $Cl^-$ channel activity. $Cl^-$ channel activity is evaluated taking the $Cl^-$ channel activity when ionomycin is added in the absence of the test compound as 100%; and the $Cl^-$ channel activity when ionomycin is not added in the absence of the test compound as 0%. Then, compounds having $Cl^-$ ion channel inhibitory effect are selected.

## INDUSTRIAL APPLICABILITY

The protein having an amino acid sequence identical or substantially identical with the amino acid sequence represented by SEQ ID NO: 1 is a diagnostic marker for pulmonary/thoracic diseases and respiratory diseases accompanied by lung/airway inflammation [e.g. chronic obstructive pulmonary diseases (such as chronic bronchitis, pulmonary emphysema, diffuse panbronchiolitis, intrinsic asthma), bronchial asthma, cystis fibrosis, hypersensitivity pneumonitis, pulmonary fibrosis, etc.], inflammatory bowel diseases, allergic conjunctivitis, and so forth. Therefore, a compound or a salt thereof that inhibits the activity of that protein, or a neutralizing antibody that inhibits the activity of that protein may be used as a prophylactic and/or therapeutic agent for various diseases, for example, pulmonary/thoracic diseases and respiratory diseases accompanied by pulmonary/airway inflammation [e.g. chronic obstructive pulmonary diseases (such as chronic bronchitis, pulmonary emphysema, diffuse panbronchiolitis, intrinsic asthma), bronchial asthma, cystis fibrosis, hypersensitivity pneumonitis, pulmonary fibrosis, etc.], inflammatory bowel diseases, allergic conjunctivitis, and so on.

The antisense polynucleotide of the present invention is capable of inhibiting the expression of the protein having an amino acid sequence identical or substantially identical with the amino acid sequence represented by SEQ ID NO: 1, and thus may be used as a prophylactic and/or therapeutic agent for various diseases, for example, pulmonary/thoracic diseases and respiratory diseases accompanied by lung/airway inflammation [e.g. chronic obstructive pulmonary diseases (such as chronic bronchitis, pulmonary emphysema, diffuse panbronchiolitis, intrinsic asthma), bronchial asthma, cystis fibrosis, hypersensitivity pneumonitis, pulmonary fibrosis, etc.], inflammatory bowel diseases, allergic conjunctivitis, and so on. Further, the various nucleotides of the present invention are useful in diagnosis, prevention or treatment of various diseases, for example, pulmonary/thoracic diseases and respiratory diseases accompanied by lung/airway inflammation [e.g. chronic obstructive pulmonary diseases (such as chronic bronchitis, pulmonary emphysema, diffuse panbronchiolitis, intrinsic asthma), bronchial asthma, cystis fibrosis, hypersensitivity pneumonitis, pulmonary fibrosis, etc.], inflammatory bowel diseases, allergic conjunctivitis, and so on.

## Claims

**1.** A method of screening for a compound or a salt thereof that inhibits the activity of a protein comprising the same or substantially the same amino acid sequence as represented by SEQ ID NO: 1, a partial peptide thereof, or a salt thereof, which comprises using a protein comprising the same or substantially the same amino acid sequence as represented by SEQ ID NO: 1, a partial peptide thereof, or a salt thereof.

**2.** A kit for screening a compound or a salt thereof that inhibits the activity of a protein comprising the same or substantially the same amino acid sequence as represented by SEQ ID NO: 1, a partial peptide thereof, or a salt thereof, which contains a protein comprising the same or substantially the same amino acid sequence as represented by SEQ ID NO: 1, a partial peptide thereof, or a salt thereof.

**3.** A compound or a salt thereof that inhibits the activity of a protein comprising the same or substantially the same amino acid sequence as represented by SEQ ID NO: 1, a partial peptide thereof, or a salt thereof, wherein said compound or salt thereof is obtainable by using the screening method according to claim 1 or the screening kit according to claim 2.

**4.** A medicine comprising the compound or salt thereof according to claim 3.

**5.** The medicine according to claim 4, which is a prophylactic and/or therapeutic agent for respiratory diseases.

**6.** The medicine according to claim 5, wherein the respiratory disease is a chronic obstructive pulmonary disease or bronchial asthma.

**7.** A method of screening for a compound or a salt thereof that inhibits the expression of the gene for a protein comprising the same or substantially the same amino acid sequence as represented by SEQ ID NO: 1, which comprises using a polynucleotide comprising a polynucleotide encoding said protein or a partial peptide thereof.

**8.** A screening kit for a compound or a salt thereof that inhibits the expression of the gene for a protein comprising the same or substantially the same amino acid sequence as represented by SEQ ID NO: 1, which contains a polynucleotide comprising a polynucleotide encoding said protein or a partial peptide thereof.

**9.** A compound or a salt thereof that inhibits the expression of the gene for a protein comprising the same or substantially the same amino acid sequence as represented by SEQ ID NO: 1, wherein said compound or salt thereof is obtainable by using the screening method according to claim 7 or the screening kit according to claim 8.

**10.** A medicine comprising the compound or salt thereof according to claim 9.

**11.** The medicine according to claim 10, which is a prophylactic and/or therapeutic agent for respiratory diseases.

**12.** The medicine according to claim 11, wherein the respiratory disease is a chronic obstructive pulmonary disease or bronchial asthma.

**13.** An antisense polynucleotide comprising a nucleotide sequence, or a part thereof, that is complementary or substantially complementary to the nucleotide sequence of a DNA encoding a protein comprising the same or substantially the same amino acid sequence as represented by SEQ ID NO: 1 or a partial peptide of said protein.

**14.** A medicine comprising the antisense polynucleotide according to claim 13.

**15.** The medicine according to claim 14, which is a prophylactic and/or therapeutic agent for respiratory diseases.

**16.** The medicine according to claim 15, wherein the respiratory disease is a chronic obstructive pulmonary disease or bronchial asthma.

**17.** An antibody to a protein comprising the same or substantially the same amino acid sequence as represented by SEQ ID NO: 1, a partial peptide thereof, or a salt thereof.

**18.** A method of quantitatively determining a protein comprising the same or substantially the same amino acid sequence as represented by SEQ ID NO: 1, a partial peptide thereof, or a salt thereof, which comprises using the antibody according to claim 17.

**19.** A medicine comprising the antibody according to claim 17.

**20.** A diagnostic agent comprising the antibody according to claim 14.

**21.** The medicine according to claim 19, which is a prophylactic and/or therapeutic agent for respiratory diseases.

**22.** The medicine according to claim 21, wherein the respiratory disease is a chronic obstructive pulmonary disease or bronchial asthma.

**23.** The diagnostic agent according to claim 20, which is a diagnostic agent for respiratory diseases.

**24.** The diagnostic agent according to claim 23, wherein the respiratory disease is a chronic obstructive pulmonary disease or bronchial asthma.

**25.** A method of diagnosing respiratory diseases, comprising using the quantitative determination method according to claim 18.

**26.** A method of screening for a compound or a salt thereof that inhibits the production of a protein comprising the same or substantially the same amino acid sequence as represented by SEQ ID NO: 1, which comprises using the antibody according to claim 17.

**27.** A kit for screening for a compound or a salt thereof that inhibits the production of a protein comprising the same or substantially the same amino acid sequence as represented by SEQ ID NO: 1, which contains the antibody according to claim 17.

**28.** A compound or a salt thereof that inhibits the production of a protein comprising the same or substantially the same amino acid sequence as represented by SEQ ID NO: 1, wherein said compound or salt thereof is obtainable by using the screening method according to claim 26 or the screening kit according to claim 27.

**29.** A medicine comprising the compound or salt thereof according to claim 28.

**30.** The medicine according to claim 29, which is a prophylactic and/or therapeutic agent for respiratory diseases.

**31.** The medicine according to claim 30, wherein the respiratory disease is a chronic obstructive pulmonary disease or bronchial asthma.

**32.** A diagnostic agent comprising a DNA encoding a protein comprising the same or substantially the same amino acid sequence as represented by SEQ ID NO: 1 or a partial peptide thereof.

**33.** The diagnostic agent according to claim 32, which is a diagnostic agent for respiratory diseases.

**34.** The diagnostic agent according to claim 33, wherein the respiratory disease is a chronic obstructive pulmonary disease or bronchial asthma.

**35.** A prophylactic and/or therapeutic agent for respiratory diseases, comprising a compound or a salt thereof that inhibits the activity of a protein comprising the same or substantially the same amino acid sequence as represented by SEQ ID NO: 1, a partial peptide thereof, or a salt thereof; a compound or a salt thereof that inhibits the expression of the gene for said protein; or a compound or a salt thereof that inhibits the production of said protein.

**36.** A method of preventing and/or treating a respiratory disease, comprising administering to a mammal an effective amount of the compound or salt thereof according to claim 3, 9 or 28.

**37.** A method of preventing and/or treating a respiratory disease, comprising administering to a mammal an effective amount of the antisense polynucleotide according to claim 13.

**38.** A method of preventing and/or treating a respiratory disease, comprising administering to a mammal an effective amount of the antibody according to claim 17.

**39.** A method of preventing and/or treating a respiratory disease, comprising administering to a mammal an effective amount of a compound or a salt thereof that inhibits the activity of a protein comprising the same or substantially the same amino acid sequence as represented by SEQ ID NO: 1, a partial peptide thereof, or a salt thereof; a compound or a salt thereof that inhibits the expression of the gene for said protein; or a compound or a salt thereof that inhibits the production of said protein.

**40.** Use of the compound or salt thereof according to claim 3, 9 or 28 for producing a prophylactic and/or therapeutic agent for respiratory diseases.

**41.** Use of the antisense polynucleotide according to claim 13 or a salt thereof for producing a prophylactic and/or therapeutic agent for respiratory diseases.

**42.** Use of the antibody according to claim 17 for producing a prophylactic and/or therapeutic agent for respiratory diseases.

**43.** Use of a compound or a salt thereof that inhibits the activity of a protein comprising the same or substantially the same amino acid sequence as represented by SEQ ID NO: 1, a partial peptide thereof, or a salt thereof; a compound or a salt thereof that inhibits the expression of the gene for said protein; or a compound or a salt thereof that inhibits the production of said protein; for producing a prophylactic and/or therapeutic agent for respiratory diseases.

Fig. 1

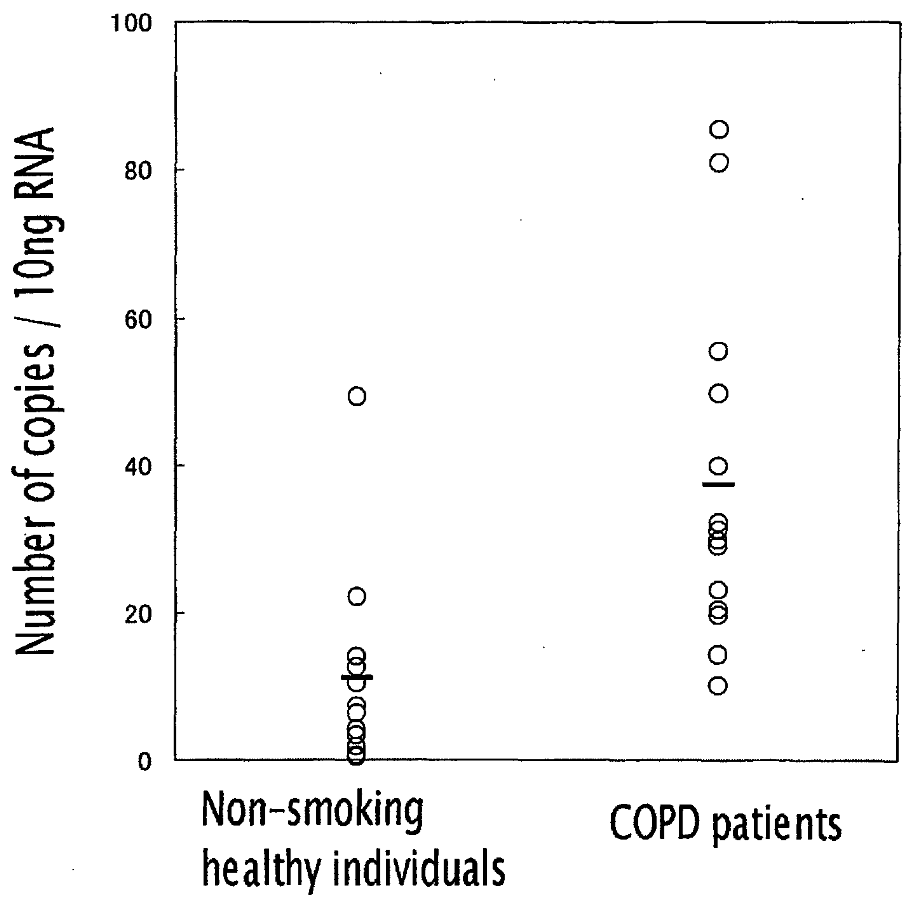
100
80
60
40
20
0
Number of copies / 10ng RNA
Non-smoking healthy individuals
COPD patients

Fig. 2

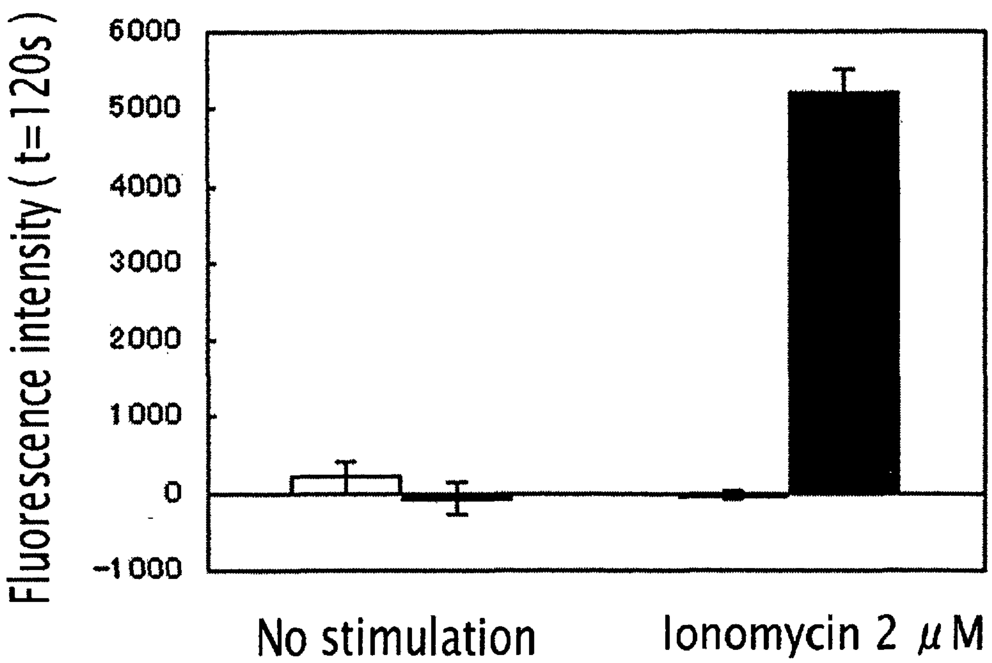
Fluorescence intensity ( t=120s )
6000
5000
4000
3000
2000
1000
0
-1000
No stimulation
Ionomycin 2 μM

Fig. 3

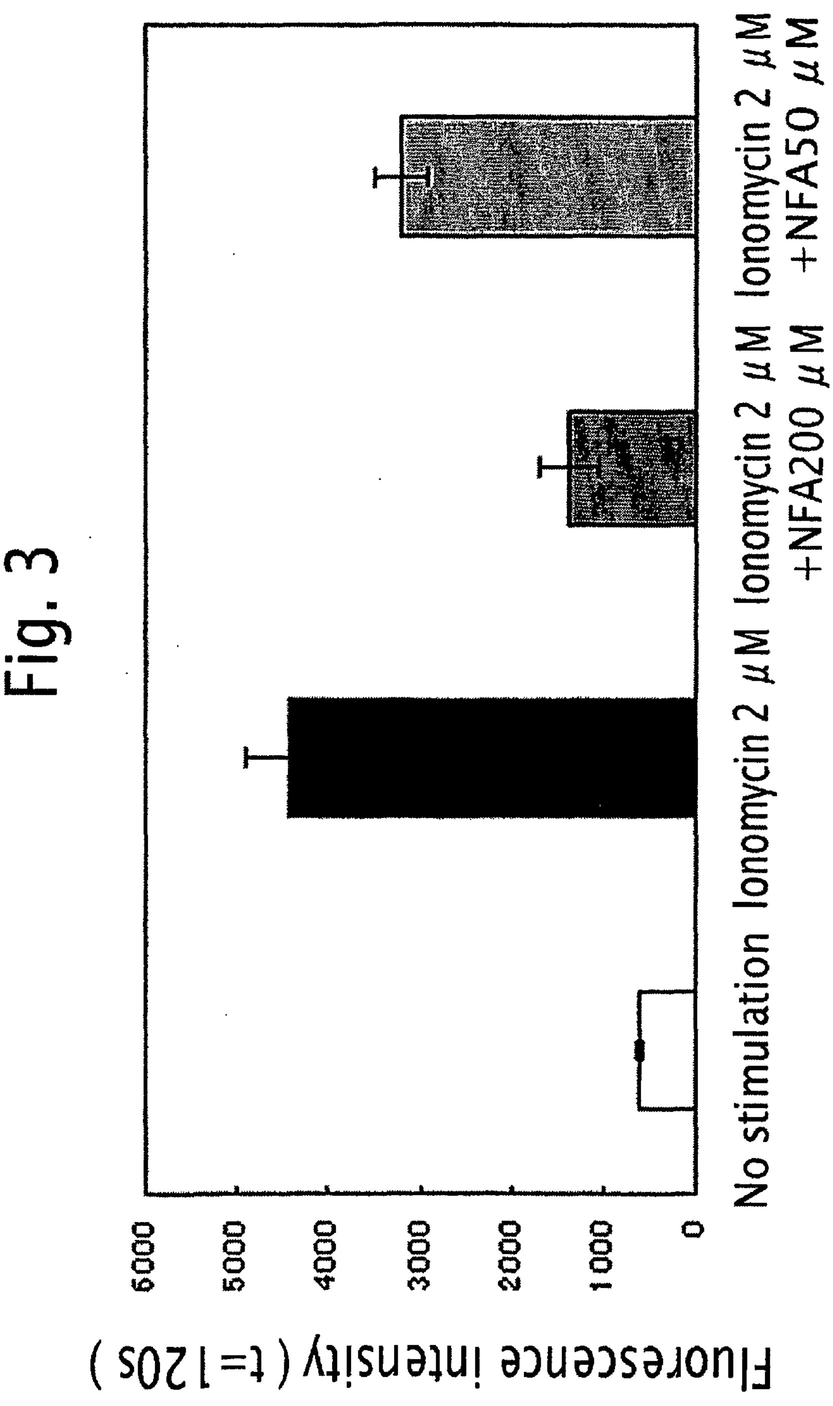
Fluorescence intensity ( t=120s )
6000
5000
4000
3000
2000
1000
0
No stimulation
Ionomycin 2 μM
Ionomycin 2 μM +NFA200 μM
Ionomycin 2 μM +NFA50 μM

## INTERNATIONAL SEARCH REPORT

International application No.
PCT/JP02/06730

A. CLASSIFICATION OF SUBJECT MATTER
Int.Cl[7] G01N33/50, G01N33/15, C12N15/09, C07K14/47, A61K38/00, A61K39/395, A61K45/00, A61P11/00, A61P11/06

According to International Patent Classification (IPC) or to both national classification and IPC

B. FIELDS SEARCHED

Minimum documentation searched (classification system followed by classification symbols)
Int.Cl[7] G01N33/50, G01N33/15, C12N15/09, C07K14/47, A61K38/00, A61K39/395, A61K45/00, A61P11/00, A61P11/06

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2002 |
| Kokai Jitsuyo Shinan Koho | 1971-2002 | Jitsuyo Shinan Toroku Koho | 1996-2002 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | WO 99/44620 A (Magainin Pharmaceuticals, Inc.), 10 September, 1999 (10.09.99), & AU 9929819 A & EP 1067940 A & JP 2002-505095 A | 1-24,26-35, 39-43 |
| A | WO 01/38530 A (Takeda Chemical Industries, Ltd.), 31 May, 2001 (31.05.01), & AU 200115484 A & JP 2002-010791 A | 1-24,26-35, 39-43 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 August, 2002 (07.08.02) | 20 August, 2002 (20.08.02) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/06730

**Box I Observations where certain claims were found unsearchable (Continuation of item 1 of first sheet)**

This international search report has not been established in respect of certain claims under Article 17(2)(a) for the following reasons:

1. [x] Claims Nos.: 25, 36-38
because they relate to subject matter not required to be searched by this Authority, namely:
The invention as set forth in claim 25 is recognized as corresponding to diagnostic methods practiced on the human body. The inventions as set forth in claims 36 to 38 are recognized as involving methods of treating mammals including human beings by therapy. Thus, (continued to extra sheet)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)

**INTERNATIONAL SEARCH REPORT**

International application No.
PCT/JP02/06730

Continuation of Box No.I-1 of continuation of first sheet(1)

the inventions as set forth in the above claims relate to a subject matter which this International Searching Authority is not required, under the provisions of Article 17(2)(a)(i) of the PCT and Rule 39.1(v) of the Regulations under the PCT, to search.

Form PCT/ISA/210 (extra sheet) (July 1998)

Sequence Listing

<110> Takeda Chemical Industries, Ltd.

<120> Use of Disease-Related Gene

<130> P02-0084PCT

<150> JP 2001-203036
<151> 2001-07-04

<160> 20

<210> 1
<211> 917
<212> PRT
<213> Homo sapiens

<400> 1
Met Gly Leu Phe Arg Gly Phe Val Phe Leu Leu Val Leu Cys Leu Leu
5 10 15
His Gln Ser Asn Thr Ser Phe Ile Lys Leu Asn Asn Asn Gly Phe Glu
20 25 30
Asp Ile Val Ile Val Ile Asp Pro Ser Val Pro Glu Asp Glu Lys Ile
35 40 45
Ile Glu Gln Ile Glu Asp Met Val Thr Thr Ala Ser Thr Tyr Leu Phe
50 55 60
Glu Ala Thr Glu Lys Arg Phe Phe Phe Lys Asn Val Ser Ile Leu Ile
65 70 75 80
Pro Glu Asn Trp Lys Glu Asn Pro Gln Tyr Lys Arg Pro Lys His Glu
85 90 95
Asn His Lys His Ala Asp Val Ile Val Ala Pro Pro Thr Leu Pro Gly
100 105 110
Arg Asp Glu Pro Tyr Thr Lys Gln Phe Thr Glu Cys Gly Glu Lys Gly
115 120 125
Glu Tyr Ile His Phe Thr Pro Asp Leu Leu Leu Gly Lys Lys Gln Asn
130 135 140
Glu Tyr Gly Pro Pro Gly Lys Leu Phe Val His Glu Trp Ala His Leu
145 150 155 160
Arg Trp Gly Val Phe Asp Glu Tyr Asn Glu Asp Gln Pro Phe Tyr Arg
165 170 175
Ala Lys Ser Lys Lys Ile Glu Ala Thr Arg Cys Ser Ala Gly Ile Ser
180 185 190
Gly Arg Asn Arg Val Tyr Lys Cys Gln Gly Gly Ser Cys Leu Ser Arg
195 200 205
Ala Cys Arg Ile Asp Ser Thr Thr Lys Leu Tyr Gly Lys Asp Cys Gln
210 215 220
Phe Phe Pro Asp Lys Val Gln Thr Glu Lys Ala Ser Ile Met Phe Met
225 230 235 240
Gln Ser Ile Asp Ser Val Val Glu Phe Cys Asn Glu Lys Thr His Asn
245 250 255
Gln Glu Ala Pro Ser Leu Gln Asn Ile Lys Cys Asn Phe Arg Ser Thr
260 265 270
Trp Glu Val Ile Ser Asn Ser Glu Asp Phe Lys Asn Thr Ile Pro Met
275 280 285
Val Thr Pro Pro Pro Pro Pro Val Phe Ser Leu Leu Lys Ile Arg Gln
290 295 300
Arg Ile Val Cys Leu Val Leu Asp Lys Ser Gly Ser Met Gly Gly Lys
305 310 315 320
Asp Arg Leu Asn Arg Met Asn Gln Ala Ala Lys His Phe Leu Leu Gln
325 330 335
Thr Val Glu Asn Gly Ser Trp Val Gly Met Val His Phe Asp Ser Thr
340 345 350
Ala Thr Ile Val Asn Lys Leu Ile Gln Ile Lys Ser Ser Asp Glu Arg
355 360 365
Asn Thr Leu Met Ala Gly Leu Pro Thr Tyr Pro Leu Gly Gly Thr Ser
370 375 380
Ile Cys Ser Gly Ile Lys Tyr Ala Phe Gln Val Ile Gly Glu Leu His

```
385                 390                 395                 400
Ser Gln Leu Asp Gly Ser Glu Val Leu Leu Leu Thr Asp Gly Glu Asp
                405                 410                 415
Asn Thr Ala Ser Ser Cys Ile Asp Glu Val Lys Gln Ser Gly Ala Ile
            420                 425                 430
Val His Phe Ile Ala Leu Gly Arg Ala Ala Asp Glu Ala Val Ile Glu
        435                 440                 445
Met Ser Lys Ile Thr Gly Gly Ser His Phe Tyr Val Ser Asp Glu Ala
    450                 455                 460
Gln Asn Asn Gly Leu Ile Asp Ala Phe Gly Ala Leu Thr Ser Gly Asn
465                 470                 475                 480
Thr Asp Leu Ser Gln Lys Ser Leu Gln Leu Glu Ser Lys Gly Leu Thr
                485                 490                 495
Leu Asn Ser Asn Ala Trp Met Asn Asp Thr Val Ile Ile Asp Ser Thr
            500                 505                 510
Val Gly Lys Asp Thr Phe Phe Leu Ile Thr Trp Asn Ser Leu Pro Pro
        515                 520                 525
Ser Ile Ser Leu Trp Asp Pro Ser Gly Thr Ile Met Glu Asn Phe Thr
    530                 535                 540
Val Asp Ala Thr Ser Lys Met Ala Tyr Leu Ser Ile Pro Gly Thr Ala
545                 550                 555                 560
Lys Val Gly Thr Trp Ala Tyr Asn Leu Gln Ala Lys Ala Asn Pro Glu
                565                 570                 575
Thr Leu Thr Ile Thr Val Thr Ser Arg Ala Ala Asn Ser Ser Val Pro
            580                 585                 590
Pro Ile Thr Val Asn Ala Lys Met Asn Lys Asp Val Asn Ser Phe Pro
        595                 600                 605
Ser Pro Met Ile Val Tyr Ala Glu Ile Leu Gln Gly Tyr Val Pro Val
    610                 615                 620
Leu Gly Ala Asn Val Thr Ala Phe Ile Glu Ser Gln Asn Gly His Thr
625                 630                 635                 640
Glu Val Leu Glu Leu Leu Asp Asn Gly Ala Gly Ala Asp Ser Phe Lys
                645                 650                 655
Asn Asp Gly Val Tyr Ser Arg Tyr Phe Thr Ala Tyr Thr Glu Asn Gly
            660                 665                 670
Arg Tyr Ser Leu Lys Val Arg Ala His Gly Gly Ala Asn Thr Ala Arg
        675                 680                 685
Leu Lys Leu Arg Pro Pro Leu Asn Arg Ala Ala Tyr Ile Pro Gly Trp
    690                 695                 700
Val Val Asn Gly Glu Ile Glu Ala Asn Pro Pro Arg Pro Glu Ile Asp
705                 710                 715                 720
Glu Asp Thr Gln Thr Thr Leu Glu Asp Phe Ser Arg Thr Ala Ser Gly
                725                 730                 735
Gly Ala Phe Val Val Ser Gln Val Pro Ser Leu Pro Leu Pro Asp Gln
            740                 745                 750
Tyr Pro Pro Ser Gln Ile Thr Asp Leu Asp Ala Thr Val His Glu Asp
        755                 760                 765
Lys Ile Ile Leu Thr Trp Thr Ala Pro Gly Asp Asn Phe Asp Val Gly
    770                 775                 780
Lys Val Gln Arg Tyr Ile Ile Arg Ile Ser Ala Ser Ile Leu Asp Leu
785                 790                 795                 800
Arg Asp Ser Phe Asp Asp Ala Leu Gln Val Asn Thr Thr Asp Leu Ser
                805                 810                 815
Pro Lys Glu Ala Asn Ser Lys Glu Ser Phe Ala Phe Lys Pro Glu Asn
            820                 825                 830
Ile Ser Glu Glu Asn Ala Thr His Ile Phe Ile Ala Ile Lys Ser Ile
        835                 840                 845
Asp Lys Ser Asn Leu Thr Ser Lys Val Ser Asn Ile Ala Gln Val Thr
    850                 855                 860
Leu Phe Ile Pro Gln Ala Asn Pro Asp Asp Ile Asp Pro Thr Pro Thr
865                 870                 875                 880
Pro Thr Pro Thr Pro Asp Lys Ser His Asn Ser Gly Val Asn Ile Ser
                885                 890                 895
Thr Leu Val Leu Ser Val Ile Gly Ser Val Val Ile Val Asn Phe Ile
            900                 905                 910
Leu Ser Thr Thr Ile
        915
```

<210> 2
<211> 2751
<212> DNA
<213> Homo sapiens

<400> 2
atggggttat tcagaggttt tgttttcctc ttagttctgt gcctgctgca ccagtcaaat 60
acttccttca ttaagctgaa taataatggc tttgaagata ttgtcattgt tatagatcct 120
agtgtgccag aagatgaaaa aataattgaa caaatagagg atatggtgac tacagcttct 180
acgtacctgt ttgaagccac agaaaaaaga tttttttca aaaatgtatc tatattaatt 240
cctgagaatt ggaaggaaaa tcctcagtac aaaaggccaa aacatgaaaa ccataaacat 300
gctgatgtta tagttgcacc acctacactc ccaggtagag atgaaccata caccaagcag 360
ttcacagaat gtggagagaa aggcgaatac attcacttca cccctgacct tctacttgga 420
aaaaaacaaa atgaatatgg accaccaggc aaactgtttg tccatgagtg ggctcacctc 480
cggtggggag tgtttgatga gtacaatgaa gatcagcctt tctaccgtgc taagtcaaaa 540
aaaatcgaag caacaaggtg ttccgcaggt atctctggta gaaatagagt ttataagtgt 600
caaggaggca gctgtcttag tagagcatgc agaattgatt ctacaacaaa actgtatgga 660
aaagattgtc aattctttcc tgataaagta caaacagaaa aagcatccat aatgtttatg 720
caaagtattg attctgttgt tgaattttgt aacgaaaaaa cccataatca agaagctcca 780
agcctacaaa acataaagtg caattttaga agtacatggg aggtgattag caattctgag 840
gattttaaaa acaccatacc catggtgaca ccacctcctc cacctgtctt ctcattgctg 900
aagatccgtc aaagaattgt gtgcttagtt cttgataagt ctggaagcat gggggggtaag 960
gaccgcctaa atcgaatgaa tcaagcagca aaacatttcc tgctgcagac tgttgaaaat 1020
ggatcctggg tggggatggt tcactttgat agtactgcca ctattgtaaa taagctaatc 1080
caaataaaaa gcagtgatga aagaaacaca ctcatggcag gattacctac atatcctctg 1140
ggaggaactt ccatctgctc tggaattaaa tatgcatttc aggtgattgg agagctacat 1200
tcccaactcg atggatccga agtactgctg ctgactgatg gggaggataa cactgcaagt 1260
tcttgtattg atgaagtgaa acaaagtggg gccattgttc attttattgc tttgggaaga 1320
gctgctgatg aagcagtaat agagatgagc aagataacag gaggaagtca tttttatgtt 1380
tcagatgaag ctcagaacaa tggcctcatt gatgcttttg ggctcttac atcaggaaat 1440
actgatctct cccagaagtc ccttcagctc gaaagtaagg gattaacact gaatagtaat 1500
gcctggatga acgacactgt cataattgat agtacagtgg gaaaggacac gttctttctc 1560
atcacatgga acagtctgcc tcccagtatt tctctctggg atcccagtgg aacaataatg 1620
gaaaatttca cagtggatgc aacttccaaa atggcctatc tcagtattcc aggaactgca 1680
aaggtgggca cttgggcata caatcttcaa gccaaagcga acccagaaac attaactatt 1740
acagtaactt ctcgagcagc aaattcttct gtgcctccaa tcacagtgaa tgctaaaatg 1800
aataaggacg taaacagttt ccccagccca atgattgttt acgcagaaat tctacaagga 1860
tatgtacctg ttcttggagc caatgtgact gctttcattg aatcacagaa tggacataca 1920
gaagttttgg aacttttgga taatggtgca ggcgctgatt ctttcaagaa tgatggagtc 1980
tactccaggt attttacagc atatacagaa aatggcagat atagcttaaa agttcgggct 2040
catggaggag caaacactgc caggctaaaa ttacggcctc cactgaatag agccgcgtac 2100
ataccaggct gggtagtgaa cggggaaatt gaagcaaacc cgccaagacc tgaaattgat 2160
gaggatactc agaccacctt ggaggatttc agccgaacag catccggagg tgcatttgtg 2220
gtatcacaag tcccaagcct tcccttgcct gaccaatacc caccaagtca aatcacagac 2280
cttgatgcca cagttcatga ggataagatt attcttacat ggacagcacc aggagataat 2340
tttgatgttg gaaaagttca acgttatatc ataagaataa gtgcaagtat tcttgatcta 2400
agagacagtt ttgatgatgc tcttcaagta aatactactg atctgtcacc aaaggaggcc 2460
aactccaagg aaagctttgc atttaaacca gaaaatatct cagaagaaaa tgcaacccac 2520
atatttattg ccattaaaag tatagataaa agcaatttga catcaaaagt atccaacatt 2580
gcacaagtaa ctttgtttat ccctcaagca aatcctgatg acattgatcc tactcctact 2640
cctactccta ctcctgataa aagtcataat tctggagtta atatttctac gctggtattg 2700
tctgtgattg ggtctgttgt aattgttaac tttattttaa gtaccaccat t 2751

<210> 3
<211> 19
<212> DNA
<213> Artificial Sequence

<220>
<223> Primer

<400> 3
aacccgccaa gacctgaaa 19

<210> 4
<211> 21
<212> DNA
<213> Artificial Sequence

<220>
<223> Primer

<400> 4
ataccacaaa tgcacctccg g 21

<210> 5
<211> 27
<212> DNA
<213> Artificial Sequence

<220>
<223> TaqMan Probe used in Example 1

<400> 5
accttggagg atttcagccg aacagca 27

<210> 6
<211> 3204
<212> DNA
<213> Homo sapiens

<400> 6
ttgagccagg aataactaga gaggaacaat ggggttattc agaggttttg ttttcctctt 60
agttctgtgc ctgctgcacc agtcaaatac ttccttcatt aagctgaata ataatggctt 120
tgaagatatt gtcattgtta tagatcctag tgtgccagaa gatgaaaaaa taattgaaca 180
aatagaggat atggtgacta cagcttctac gtacctgttt gaagccacag aaaaaagatt 240
tttttcaaa aatgtatcta tattaattcc tgagaattgg aaggaaaatc ctcagtacaa 300
aaggccaaaa catgaaaacc ataaacatgc tgatgttata gttgcaccac ctacactccc 360
aggtagagat gaaccataca ccaagcagtt cacagaatgt ggagagaaag gcgaatacat 420
tcacttcacc cctgaccttc tacttggaaa aaaacaaaat gaatatggac caccaggcaa 480
actgtttgtc catgagtggg ctcacctccg gtggggagtg tttgatgagt acaatgaaga 540
tcagcctttc taccgtgcta agtcaaaaaa aatcgaagca acaaggtgtt ccgcaggtat 600
ctctggtaga aatagagttt ataagtgtca aggaggcagc tgtcttagta gagcatgcag 660
aattgattct acaacaaaac tgtatggaaa agattgtcaa ttctttcctg ataaagtaca 720
aacagaaaaa gcatccataa tgtttatgca aagtattgat tctgttgttg aattttgtaa 780
cgaaaaaacc cataatcaag aagctccaag cctacaaaac ataaagtgca attttagaag 840
tacatgggag gtgattagca attctgagga ttttaaaaac accataccca tggtgacacc 900
acctcctcca cctgtcttct cattgctgaa gatcagtcaa agaattgtgt gcttagttct 960
tgataagtct ggaagcatgg ggggtaagga ccgcctaaat cgaatgaatc aagcagcaaa 1020
acatttcctg ctgcagactg ttgaaaatgg atcctgggtg gggatggttc actttgatag 1080
tactgccact attgtaaata agctaatcca aataaaaagc agtgatgaaa gaaacacact 1140
catggcagga ttacctacat atcctctggg aggaacttcc atctgctctg gaattaaata 1200
tgcatttcag gtgattggag agctacattc ccaactcgat ggatccgaag tactgctgct 1260
gactgatggg gaggataaca ctgcaagttc ttgtattgat gaagtgaaac aaagtggggc 1320
cattgttcat tttattgctt tgggaagagc tgctgatgaa gcagtaatag agatgagcaa 1380
gataacagga ggaagtcatt tttatgtttc agatgaagct cagaacaatg gcctcattga 1440
tgcttttggg gctcttacat caggaaatac tgatctctcc cagaagtccc ttcagctcga 1500
aagtaaggga ttaacactga atagtaatgc ctggatgaac gacactgtca taattgatag 1560
tacagtggga aaggacacgt tctttctcat cacatggaac agtctgcctc ccagtatttc 1620
tctctgggat cccagtggaa caataatgga aaatttcaca gtggatgcaa cttccaaaat 1680
ggcctatctc agtattccag gaactgcaaa ggtgggcact tgggcataca atcttcaagc 1740
caaagcgaac ccagaaacat taactattac agtaacttct cgagcagcaa attcttctgt 1800
gcctccaatc acagtgaatg ctaaaatgaa taaggacgta aacagtttcc ccagcccaat 1860
gattgtttac gcagaaattc tacaaggata tgtacctgtt cttggagcca atgtgactgc 1920
tttcattgaa tcacagaatg gacatacaga agttttggaa cttttggata atggtgcagg 1980
cgctgattct ttcaagaatg atggagtcta ctccaggtat tttacagcat atacagaaaa 2040
tggcagatat agcttaaaag ttcgggctca tggaggagca aacactgcca ggctaaaatt 2100
acggcctcca ctgaatagag ccgcgtacat accaggctgg gtagtgaacg gggaaattga 2160
agcaaacccg ccaagacctg aaattgatga ggatactcag accaccttgg aggatttcag 2220
ccgaacagca tccggaggtg catttgtggt atcacaagtc ccaagccttc ccttgcctga 2280
ccaataccca ccaagtcaaa tcacagacct tgatgccaca gttcatgagg ataagattat 2340
tcttacatgg acagcaccag gagataattt tgatgttgga aaagttcaac gttatatcat 2400
aagaataagt gcaagtattc ttgatctaag agacagtttt gatgatgctc ttcaagtaaa 2460
tactactgat ctgtcaccaa aggaggccaa ctccaaggaa agctttgcat ttaaaccaga 2520
aaatatctca gaagaaaatg caacccacat atttattgcc attaaaagta tagataaaag 2580
caatttgaca tcaaaagtat ccaacattgc acaagtaact ttgtttatcc ctcaagcaaa 2640

tcctgatgac attgatccta ctcctactcc tactcctact cctgataaaa gtcataattc 2700
tggagttaat atttctacgc tggtattgtc tgtgattggg tctgttgtaa ttgttaactt 2760
tattttaagt accaccattt gaaccttaac gaagaaaaaa atcttcaagt agacctagaa 2820
gagagtttta aaaaacaaaa caatgtaagt aaaggatatt tctgaatctt aaaattcatc 2880
ccatgtgtga tcataaactc ataaaaataa ttttaagatg tcggaaaagg atactttgat 2940
taaataaaaa cactcatgga tatgtaaaaa ctgtcaagat taaaatttaa tagtttcatt 3000
tatttgttat tttatttgta agaaatagtg atgaacaaag atcctttttc atactgatac 3060
ctggttgtat attatttgat gcaacagttt tctgaaatga tatttcaaat tgcatcaaga 3120
aattaaaatc atctatctga gtagtcaaaa tacaagtaaa ggagagcaaa taaacaacat 3180
ttggaaaaaa aaaaaaaaaa aaaa 3204

<210> 7
<211> 24
<212> DNA
<213> Artificial Sequence

<220>
<223> Primer

<400> 7
ggaataacta gagaggaaca atgg 24

<210> 8
<211> 24
<212> DNA
<213> Artificial Sequence

<220>
<223> Primer

<400> 8
actctcttct aggtctactt gaag 24

<210> 9
<211> 17
<212> DNA
<213> Artificial Sequence

<220>
<223> Primer

<400> 9
caggaaacag ctatgac 17

<210> 10
<211> 20
<212> DNA
<213> Artificial Sequence

<220>
<223> Primer

<400> 10
taatacgact cactataggg 20

<210> 11
<211> 21
<212> DNA
<213> Artificial Sequence

<220>
<223> Primer

<400> 11
actcccaggt agagatgaac c 21

<210> 12
<211> 22

<212> DNA
<213> Artificial Sequence

<220>
<223> Primer

<400> 12
tcaagaagct ccaagcctac aa 22

<210> 13
<211> 21
<212> DNA
<213> Artificial Sequence

<220>
<223> Primer

<400> 13
tacatatcct ctgggaggaa c 21

<210> 14
<211> 21
<212> DNA
<213> Artificial Sequence

<220>
<223> Primer

<400> 14
actgatctct cccagaagtc c 21

<210> 15
<211> 23
<212> DNA
<213> Artificial Sequence

<220>
<223> Primer

<400> 15
gaaatactgg gaggcagact gtt 23

<210> 16
<211> 20
<212> DNA
<213> Artificial Sequence

<220>
<223> Primer

<400> 16
ccccagccca atgattgttt 20

<210> 17
<211> 19
<212> DNA
<213> Artificial Sequence

<220>
<223> Primer

<400> 17
aacccgccaa gacctgaaa 19

<210> 18
<211> 23
<212> DNA
<213> Artificial Sequence

<220>
<223> Primer

<400> 18
ctcctggtgc tgtccatgta aga 23

<210> 19
<211> 24
<212> DNA
<213> Artificial Sequence

<220>
<223> Primer

<400> 19
ggaataacta gagaggaaca atgg 24

<210> 20
<211> 24
<212> DNA
<213> Artificial Sequence

<220>
<223> Primer

<400> 20
actctcttct aggtctactt gaag 24